# EUROPEAN PATENT SPECIFICATION

(11) **EP 1 200 114 B1**
(45) Date of publication and mention of the grant of the patent: **06.05.2009**
(21) Application number: 00956238.0
(22) Date of filing: 19.07.2000
(51) Int. Cl.: A61K 38/17, A61K 31/00, A61K 48/00, G01N 33/50, A61P 9/10, A61P 25/28, A61P 29/00, A61P 35/00

(54) **PPAR DELTA INHIBITORS FOR THE TREATMENT OF CARDIOVASCULAR DISEASES**
PPAR DELTA-INHIBITOREN ZUR BEHANDLUNG KARDIOVASCULÄRER ERKRANKUNGEN
INHIBITEURS DE PPAR-DELTA POUR LE TRAITEMENT DE MALADIES CARDIO-VASCULAIRES

(30) Priority: 23.07.1999 GB 9917405
(43) Date of publication of application: 02.05.2002
(73) Proprietor: The University of Dundee, Dundee DD1 4HN (GB)
(72) Inventor: PALMER, Colin, Neil, Alexander, The University of Dundee, Dundee Tayside DD1 9SY (GB); VOSPER, Helen The University of Dundee, Dundee Tayside DD1 9SY (GB); WOLF, Charles, Roland, The University of Dundee, Dundee Tayside DD1 9SY (GB)
(74) Representative: Rutter, Keith
(86) International application number: PCT/EP2000/006986
(87) International publication number: WO 2001/007066

(56) References cited:
- WO-A-00/32190
- WO-A-97/28149
- WO-A-98/43081
- WILLSON T M ET AL: "The PPARs: From orphan receptors to drug discovery." JOURNAL OF MEDICINAL CHEMISTRY, vol. 43, no. 4, 24 February 2000 (2000-02-24), pages 527-550, XP002158740
- PETERS J M ET AL: "Growth, adipose, brain, and skin alterations resulting from targeted disruption of the mouse peroxisome proliferator-activated receptor beta (delta)." MOLECULAR AND CELLULAR BIOLOGY, vol. 20, no. 14, July 2000 (2000-07), pages 5119-5128, XP002158741 cited in the application
- BERGER J ET AL: "Novel peroxisome proliferator-activated receptor (PPAR) gamma and PPARdelta ligands produce distinct biological effects." JOURNAL OF BIOLOGICAL CHEMISTRY, vol. 274, no. 10, 5 March 1999 (1999-03-05), pages 6718-6725, XP002158742 cited in the application
- RICOTE M ET AL: "Expression of the peroxisome proliferator-activated receptor gamma (PPARgamma) in human atherosclerosis and regulation in macrophages by colony stimulating factors and oxidized low density lipoprotein." PROCEEDINGS OF THE NATIONAL ACADEMY OF SCIENCES OF THE UNITED STATES, vol. 95, no. 13, 23 June 1998 (1998-06-23), pages 7614-7619, XP002158743
- POLLMAN M J ET AL: "Inhibition of neointimal cell bcl-x expression induces apoptosis and regression of vascular disease." NATURE MEDICINE, vol. 4, no. 2, February 1998 (1998-02), pages 222-227, XP002158744 cited in the application
- MANO H ET AL: "Cloning and function of rabbit peroxisome proliferator-activated receptor delta/beta in mature osteoclasts." JOURNAL OF BIOLOGICAL CHEMISTRY, vol. 275, no. 11, 17 March 2000 (2000-03-17), pages 8126-8132, XP002158745
- ZHOU G ET AL: "Nuclear receptors have distinct affinities for coactivators: Characterisation by fluorescence resonance energy transfer" MOLECULAR ENDOCRINOLOGY, vol. 12, no. 10, October 1998 (1998-10), pages 1594-1604, XP002915572
- KREY G ET AL: "Fatty acids, eicosanoids and hypolipidemic agents identified as ligands of peroxisome proliferator- activated receptors by coactivator- dependant receptor ligand assay" MOLECULAR ENDOCRINOLOGY, vol. 11, no. 6, June 1997 (1997-06), pages 779-791, XP002915086

## Description

The present invention relates to treatment and a target for cardiovascular drug design and compounds which are directed at this target.

Lipid homeostasis is tightly controlled during normal development and health; however, dysfunction in lipid homeostasis results in the development of diseases such as atherosclerosis, obesity, diabetes and cancer. Critical regulators in lipid homeostasis include a family of lipid sensing transcription factors known as Peroxisome Proliferator Activated Receptors (PPARs). PPARs are members of the steroid hormone/nuclear receptor subfamily that are activated by structurally diverse chemicals such as fatty acids, prostanoids, hypolipidemic drugs, non-steroidal anti-inflammatory drugs, insulin sensitizers and environmental pollutants (1,2). The first PPAR to be described, PPARα, controls lipid metabolism in the liver and is activated by lipid lowering drugs such as clofibrate (3,4). Mice that have had the gene encoding PPARα disrupted have a deficiency in their ability to metabolise and excrete hepatic lipid and the hepatocytes accumulate large lipid droplets in response to hypolipidemic drugs (4). Another isoform, PPARγ, is required for the lipid stimulated development of adipocytes. PPARγ is a high affinity nuclear receptor for prostanoids (5) and is the pharmacological target for the thiazolidinedione group of anti-diabetic agents such as rosiglitazone (BRL49653) (6), International Application Publication Number WO 94/05659 (SmithKline Beecham PLC). Genetic ablation of this gene is lethal (7).

The function of another form of PPAR, PPARδ, remains unknown. PPARδ has been reported to be expressed at very low levels in all tissues examined (8); however, we have now made the observations that PPARδ is expressed selectively in cells of the monocyte/macrophage lineage and is upregulated, along with PPARγ, during phorbol ester-induced macrophage differentiation. PPARδ is a fatty acid sensor and is activated by the unsaturated fatty acids linoleic acid and oleic acid, and these fatty acids have been implicated as being important signalling compounds in the differentiation of monocytes (9,10,11). PPARδ is not activated by fibrate drugs, such as clofibrate, or by thiazolidinediones such as BRL49653.

WO 97/28149 (Merck & Co. Inc.) describes PPARδ agonists which are purported to be useful for raising high density lipoprotein (HDL) plasma levels in mammals, and for preventing, halting or slowing the progression of atherosclerotic cardiovascular diseases.

High fat diets are known to be a major factor in the development of atherosclerosis, and many drugs that are used in the treatment of atherosclerosis modify lipid metabolism. Lipids are physically associated with the pathology of atherosclerosis in the macrophage-derived foam cell which are highly activated macrophages that are filled with lipid droplets. These foam cells are present at high concentrations in vascular lesions known as plaques, that eventually burst and result in thrombotic blockage of the blood vessels. This blockage may be clinically represented as a heart attack, stroke or peripheral artery disease. We have now shown that mouse macrophages and human THP-1 monocytic cell line express PPARδ when activated by phorbol esters. The activated THP-1 cells become adherent and spread on plastic culture dishes. In the appropriate fatty acid conditions, these cells will fill with lipid as detected by Oil Red O staining and become foam cells. These cells express genes that are associated with foam cells *in vivo,* such as IL-1β, TNFα, IGF-1, CD36, MCP-1 and thromboxane synthase. These cells provide a model of lipid-induced foam cell formation. This accumulation can be prevented by candidate anti-atherosclerosis drugs such as BRL49653, thus demonstrating an anti-atherosclerotic property of the drug and that the model is useful in identifying anti-atherosclerotic drugs. This assay can be performed in a microtitre plate format and the lipid staining quantitated optically in a microtitre plate reader.

Using a selectable expression vector we have modified THP-1 cells to express PPARδ antisense RNA. This cell line grow normally in culture, however when treated with phorbol ester this cell line no longer differentiates. Trypan blue staining of this cell line after phorbol ester treatment reveals that the cells have died. Therefore inhibition of PPARδ function has switched the response to inflammatory stimuli from differentiation to death. The atherosclerotic plaque is filled with highly activated macrophage derived foam cells, and local concentrations of inflammatory mediators is very high within the plaque. Without being bound by any theory, we believe that inhibition of PPARδ function in foam cells within, for example, atheromatous lesions will be useful to initiate a self-destruct cascade within the plaque. Controlled removal of foam cells without inflammation or necrosis has recently been demonstrated as an efficacious method of promoting plaque regression, using anti-sense therapy against BCL-X (Pollman et al (1998) Nature Medicine 4, 222-227). The targeting of PPARδ in the activated macrophage is, we believe, likely to be highly specific as it is a response that relies upon the specific phenotypic state of the activated foam cell.

The invention provides compounds which interfere with the formation of the foam cell development, or lead to the controlled removal of potential foam cells. Such compounds are believed to be useful in the promotion of plaque regression, and therefore the reduction in risk of heart disease, stroke or thrombosis.

We have shown that PPARδ fulfils a critical role in the activation of macrophages and that selective inhibition of PPARδ activity can prevent foam cell formation.

We have also shown that PPARγ agonists inhibit lipid accumulation and that PPARδ and PPARγ oppose each other in the process of foam cell formation.

A first aspect of the invention relates to preventing or reducing foam cell development from macrophages or other cells, or removing foam cells by contacting said macrophages or other cells or foam cells with an effective amount of an inhibitor of PPARδ activity.

Suitably, the macrophages or other cells are those involved in atherosclerotic pathology.

Although the described method has use in relation to studying macrophages and foam cells in culture, the method is particularly suited to preventing or reducing foam cell development, or removing foam cells, *in vivo* in particular within the body of a human or other mammal. Foam cells are lipid-laden cells such as leukocytes, usually macrophages. They have internalized lipids and store them as cytoplasmic droplets, which gives the cells their characteristic foamy appearance in light microscopy.

A second aspect of the invention relates to preventing or reducing foam cell development from macrophages or other cells, or removing foam cells, in a patient, comprising administering to the patient an effective amount of an inhibitor of PPARδ activity.

Suitably, the macrophages or other cells are those involved in atherosclerotic pathology.

Cells which may develop into foam cells include not only macrophages but also smooth muscle cells. Preferably, the inhibitor of PPARδ activity is one which prevents or reduces foam cell development from macrophages, or removes macrophage-derived foam cells. More preferably, this is can be used for preventing or reducing foam cell development from macrophages.

Without prejudice to any further aspects of the invention, and while not being bound by any theory concerning the invention, it is believed that the development of foam cells from macrophages is associated with various disease states and that an inhibitor of PPARδ activity will prevent or reduce, at least to a desirable extent the development of foam cells from macrophages. The diseases which are associated with foam cell development include but are not limited to atherosclerosis, heart disease, stroke, peripheral artery diseases, and angina.

The methods may also be used for the prevention of restenosis and for regression of atheroma.

The finding that inhibition of PPARδ activity prevents the activation of macrophages would also suggest that the methods may also be used in the treatment of inflammatory disorders. Examples of inflammatory disorders include rheumatoid arthritis, systemic sclerosis, and lupus. In support of a role for PPARδ in inflammation, the non-steroidal anti-inflammatory drug sulindac, which blocks PPARδ transcriptional activity, is unable to prevent PMA (phorbolmyristate acetate)-induced epidermal hyperplasia and inflammation in PPARδ-null mice (Peters J M et al (2000) Mol. and Cell Biol., 20, 5119-5128).

There are a wide variety of disorders that arise through the pathology of atherosclerosis and include angina, coronary heart disease, stroke, peripheral vascular disease, Alzheimer's disease, vascular dementia, systemic sclerosis and retinal vascular degeneration.

A third aspect of the invention relates preventing or treating a vascular disease associated with plaque formation and/or thrombotic blockage of the blood vessels in a patient, the method comprising administering to the patient an effective amount of an inhibitor of PPARδ activity. It is believed that the method of this invention is particularly suited to the prevention or treatment of atherosclerosis, coronary heart disease, stroke or peripheral heart disease.

We have shown that inhibition of PPARδ activity inhibits PMA-stimulated proliferation of cells. Thus, a fourth aspect of the invention relates to preventing or reducing proliferation of cells, which method comprises contacting the cells with an inhibitor of PPARδ activity. Cells which are stimulated to proliferate by PMA include skin cells, colon cells, breast cells and prostate cells. In support of the role of PPARδ in tumorigenesis it has recently been demonstrated that non-steroidal anti-inflammatory agents exert their anti cancer activity in colon-derived cells via blockade of PPARδ transcriptional activity and promoting apoptosis. (He T-C et al., 1999 Cell, 99, 335-343)

PPARδ is involved in the action of the tumour promoter, PMA.

Thus, a fifth aspect of the invention relates to treating or preventing cancer in a patient, the method comprising administering to the patient an effective amount of an inhibitor of PPARδ activity.

The method of the invention is particularly suited for use in relation to cancers of the skin.

The method of the invention is also particularly suited for use in relation to cancers of the breast.

The method of the invention is also particularly suited for use in relation to cancers of the colon.

The method of the invention is also particularly suited for use in relation to cancers of the prostate.

By "an effective amount of an inhibitor of PPARδ activity" we include the meaning an amount which is effective to prevent or reduce foam cell development from macrophages or other cells, or remove foam cells and, in particular, an amount which is effective to prevent the disease (by which we include the prevention of the progression from an asymptomatic state to a symptomatic state) or treat the disease (by which we include ameliorating the symptoms and slowing its progression) to a clinically useful extent. An extent which is clinically useful may readily be determined by the physician.

By "an inhibitor of PPARδ activity" we mean any suitable inhibitor which may reduce PPARδ activity in the cell, for example in the cell of a patient. It is particularly preferred that the inhibitor of PPARδ activity is one which inhibits PPARδ activity in a macrophage cell or a foam cell derived from a macrophage cell. The term "inhibitor of PEARδ activity" includes compounds which block the effect of PPARδ agonists.

It is preferred if the inhibitor of PPARδ activity is selective for PPARδ. In particular, it is preferred that the inhibitor is effective in inhibiting PPARδ activity but is substantially incapable of inhibiting the activity of other PPAR isoforms such as PPARα or PPARγ. Although it is preferred that the inhibitor of PPARδ activity does not modify the activity of other PPAR isoforms, it may be advantageous if the molecule is an agonist or activator of PPARγ activity. It is also preferred if the inhibitor of PPARδ activity does not modify the activity of other steroid hormone receptor family receptors. By "selective" we mean that the compound is at least ten-fold more effective in inhibiting PPARδ activity than it is in modifying the activity of another PPAR isoform. Preferably it is at least 100-fold more selective.

The inhibitor may be one which prevents the expression of PPARδ in a cell. By "prevents the expression" we mean substantially prevents expression or at least reduces the level of expression of PPARδ to a useful extent. Typically, the compound which prevents the expression of PPARδ in a cell is a nucleic-acid based molecule.

As is shown in the Examples, we have demonstrated that the use of the antisense of the entire human PPARδ coding sequence, including portions of untranslated regions, is able to inhibit foam cell formation from macrophages. It will be appreciated that smaller portions of the PPARδ cDNA may be used as antisense agents, especially those corresponding to the start of translation of the PPARδ protein.

Antisense molecules may be designed by reference to the PPARδ sequence, the human cDNA sequence of which is given in Schmidt et al (1992) Mol. Endocrinol. 6, 1634-1641 and in GenBank Accession No. L07592.

PPARδ antisense agents include agents which bind to PPARδ mRNA and, preferably, inhibits its translation. By "antisense agent" we also include nucleic acid based molecules which are able to form triplexes with PPARδ genomic sequence and prevent selectively transcription or translation of the gene.

The antisense molecule may be RNA (in which case it is transcribed from a vector designed to produce antisense transcripts) or it may be DNA or DNA-like molecules, such as antisense oligonucleotides.

It is believed that over-expression of anti-sense RNA from suitable portions of human genomic sequences corresponding to positions 67886 to 139948 of the human PAC clone No. 109F14 will be effective in inhibiting PPARδ activity. PAC Clone No. 109F14 is available from the HGMP Resource Centre, Genome Campus, Hinxton. Cambridgeshire, CB10 1SB, UK. PPARδ gene sequence has Accession No. AL022721.

Antisense oligonucleotides can be designed by reference to the PPARδ cDNA or gene sequence.

Oligonucleotides are subject to being degraded or inactivated by cellular endogenous nucleases. To counter this problem, it is possible to use modified oligonucleotides, eg having altered internucleotide linkages, in which the naturally occurring phosphodiester linkages have been replaced with another linkage. For example, Agrawal et al (1988) Proc. Natl. Acad. Sci. USA 85, 7079-7083 showed increased inhibition in tissue culture of HIV-1 using oligonucleotide phosphoramidates and phosphorothioates. Sarin et al (1988) Proc. Natl. Acad. Sci. USA 85, 7448-7451 demonstrated increased inhibition of HIV-1 using oligonucleotides methylphosphonates. Agrawal et al (1989) Proc. Natl. Acad. Sci. USA 86, 7790-7794 showed inhibition of HIV-1 replication in both early-infected and chronically infected cell cultures, using nucleotide sequence-specific oligonucleotide phosphorothioates. Leither et al (1990) Proc. Natl. Acad. Sci. USA 87, 3430-3434 report inhibition in tissue culture of influenza virus replication by oligonucleotide phosphorothioates.

Oligonucleotides having artificial linkages have been shown to be resistant to degradation *in vivo.* For example, Shaw et al (1991) in Nucleic Acids Res. 19, 747-750, report that otherwise unmodified oligonucleotides become more resistant to nucleases *in vivo* when they are blocked at the 3' end by certain capping structures and that uncapped oligonucleotide phosphorothioates are not degraded *in vivo.*

A detailed description of the H-phosphonate approach to synthesizing oligonucleoside phosphorothioates is provided in Agrawal and Tang (1990) Tetrahedron Letters 31, 7541-7544, the teachings of which are hereby incorporated herein by reference. Syntheses of oligonucleoside methylphosphonates, phosphorodithioates, phosphoramidates, phosphate esters, bridged phosphoramidates and bridge phosphorothioates are known in the art. See, for example, Agrawal and Goodchild (1987) Tetrahedron Letters 28, 3539: Nielsen et al (1988) Tetrahedron Letters 29, 2911; Jager et al (1988) Biochemistry 27, 7237; Uznanski et al (1987) Tetrahedron Letters 28, 3401; Bannwarth (1988) Helv. Chim. Acta. 71, 1517; Crosstick and Vyle (1989) Tetrahedron Letters 30, 4693; Agrawal et al (1990) Proc. Natl. Acad. Sci. USA 87, 1401-1405, the teachings of which are incorporated herein by reference. Other methods for synthesis or production also are possible. In a preferred embodiment the oligonucleotide is a deoxyribonucleic acid (DNA), although ribonucleic acid (RNA) sequences may also be synthesized and applied.

The oligonucleotides useful in the invention preferably are designed to resist degradation by endogenous nucleolytic enzymes. *In vivo* degradation of oligonucleotides produces oligonucleotide breakdown products of reduced length. Such breakdown products are more likely to engage in non-specific hybridization and are less likely to be effective, relative to their full-length counterparts. Thus, it is desirable to use oligonucleotides that are resistant to degradation in the body and which are able to reach the targeted cells. The present oligonucleotides can be rendered more resistant to degradation *in vivo* by substituting one or more internal artificial internucleotide linkages for the native phosphodiester linkages, for example, by replacing phosphate with sulphur in the linkage. Examples of linkages that may be used include phosphorothioates, methylphosphonates, sulphone, sulphate, ketyl, phosphorodithioates, various phosphoramidates, phosphate esters, bridged phosphorothioates and bridged phosphoramidates. Such examples are illustrative, rather than limiting, since other internucleotide linkages are known in the art. See, for example, Cohen, (1990) Trends in Biotechnology*.* The synthesis of oligonucleotides having one or more of these linkages substituted for the phosphodiester internucleotide linkages is well known in the art, including synthetic pathways for producing oligonucleotides having mixed internucleotide linkages.

Oligonucleotides can be made resistant to extension by endogenous enzymes by "capping" or incorporating similar groups on the 5' or 3' terminal nucleotides. A reagent for capping is commercially available as Amino-Link II^{™} from Applied BioSystems Inc, Foster City, CA. Methods for capping are described, for example, by Shaw et al (1991) Nucleic Acids Res. 19, 747-750 and Agrawal et al (1991) Proc. Natl. Acad. Sci. USA 88(17), 7595-7599, the teachings of which are hereby incorporated herein by reference.

A further method of making oligonucleotides resistant to nuclease attack is for them to be "self-stabilized" as described by Tang et al (1993) Nucl. Acids Res. 21, 2729-2735 incorporated herein by reference. Self-stabilized oligonucleotides have hairpin loop structures at their 3' ends, and show increased resistance to degradation by snake venom phosphodiesterase, DNA polymerase I and fetal bovine serum. The self-stabilized region of the oligonucleotide does not interfere in hybridization with complementary nucleic acids, and pharmacokinetic and stability studies in mice have shown increased *in vivo* persistence of self-stabilized oligonucleotides with respect to their linear counterparts.

In accordance with the invention, the inherent binding specificity of antisense oligonucleotides characteristic of base pairing is enhanced by limiting the availability of the antisense compound to its intended locus *in vivo,* permitting lower dosages to be used and minimizing systemic effects. Thus, oligonucleotides are applied locally to achieve the desired effect. The concentration of the oligonucleotides at the desired locus is much higher than if the oligonucleotides were administered systemically, and the therapeutic effect can be achieved using a significantly lower total amount. The local high concentration of oligonucleotides enhances penetration of the targeted cells and effectively blocks translation of the target nucleic acid sequences.

The oligonucleotides can be delivered to the locus by any means appropriate for localized administration of a drug. For example, a solution of the oligonucleotides can be injected directly to the site or can be delivered by infusion using an infusion pump. The oligonucleotides also can be incorporated into an implantable device which when placed at the desired site, permits the oligonucleotides to be released into the surrounding locus.

The oligonucleotides are most preferably administered *via* a hydrogel material. The hydrogel is noninflammatory and biodegradable. Many such materials now are known, including those made from natural and synthetic polymers. In a preferred embodiment, the method exploits a hydrogel which is liquid below body temperature but gels to form a shape-retaining semisolid hydrogel at or near body temperature. Preferred hydrogel are polymers of ethylene oxide-propylene oxide repeating units. The properties of the polymer are dependent on the molecular weight of the polymer and the relative percentage of polyethylene oxide and polypropylene oxide in the polymer. Preferred hydrogels contain from about 10 to about 80% by weight ethylene oxide and from about 20 to about 90% by weight propylene oxide. A particularly preferred hydrogel contains about 70% polyethylene oxide and 30% polypropylene oxide. Hydrogels which can be used are available, for example, from BASF Corp., Parsippany, NJ, under the tradename Pluronic^{™}.

In this embodiment, the hydrogel is cooled to a liquid state and the oligonucleotides are admixed into the liquid to a concentration of about 1mg oligonucleotide per gram of hydrogel. The resulting mixture then is applied onto the surface to be treated, for example by spraying or painting during surgery or using a catheter or endoscopic procedures. As the polymer warms, it solidifies to form a gel, and the oligonucleotides diffuse out of the gel into the surrounding cells over a period of time defined by the exact composition of the gel.

The oligonucleotides can be administered by means of other implants that are commercially available or described in the scientific literature, including liposomes, microcapsules and implantable devices. For example, implants made of biodegradable materials such as polyanhydrides, polyorthoesters, polylactic acid and polyglycolic acid and copolymers thereof, collagen, and protein polymers, or non-biodegradable materials such as ethylenevinyl acetate (EVAc), polyvinyl acetate, ethylene vinyl alcohol, and derivatives thereof can be used to locally deliver the oligonucleotides. The oligonucleotides can be incorporated into the material as it is polymerized or solidified, using melt or solvent evaporation techniques, or mechanically mixed with the material. In one embodiment, the oligonucleotides are mixed into or applied onto coatings for implantable devices such as dextran coated silica beads, stents, or catheters.

The dose of oligonucleotides is dependent on the size of the oligonucleotides and the purpose for which is it administered. In general, the range is calculated based on the surface area of tissue to be treated. The effective dose of oligonucleotide is somewhat dependent on the length and chemical composition of the oligonucleotide but is generally in the range of about 30 to 3000µg per square centimetre of tissue surface area.

The oligonucleotides may be administered to the patient systemically for both therapeutic and prophylactic purposes. The oligonucleotides may be administered by any effective method, for example, parenterally (eg intravenously, subcutaneously, intramuscularly) or by oral, nasal or other means which permit the oligonucleotides to access and circulate in the patient's bloodstream. Oligonucleotides administered systemically preferably are given in addition to locally administered oligonucleotides, but also have utility in the absence of local administration. A dosage in the range of from about 0.1 to about 10 grams per administration to an adult human generally will be effective for this purpose.

In addition to the antisense agents described above, ribozymes, in particular hammerhead ribozymes, can be designed based on the PPARδ cDNA or gene sequence which are believed to be useful as inhibitors of PPARδ activity.

Ribozymes which may be encoded in the nucleic acid to be delivered to the target are described in Cech and Herschlag "Site-specific cleavage of single stranded DNA" US 5,180,818; Altman *et al* "Cleavage of targeted RNA by RNAse P" US 5,168.053. Cantin *et al* "Ribozyme cleavage of HIV-1 RNA" US 5,149,796; Cech *et al* "RNA ribozyme restriction endoribonucleases and methods", US 5,116,742: Been *et al* "RNA ribozyme polymerases, dephosphorylases, restriction endonucleases and methods". US 5,093,246; and Been *et al* "RNA ribozyme polymerases, dephosphorylases, restriction endoribonucleases and methods; cleaves single-stranded RNA at specific site by transesterification". US 4,987,071, all incorporated herein by reference. Suitable targets for ribozymes include transcription factors such as c-fos and c-myc, and bcl-2. Durai et al (1997) Anticancer Res. 17, 3307-3312 describes a hammerhead ribozyme against bcl-2.

Genetic constructs which express the ribozymes or antisense compounds useful in the methods of the invention may readily be made. Suitably, the genetic constructs are ones which are adapted to deliver and express the ribozyme or antisense molecule in the target cell. Thus, the method of the invention comprises a method of gene therapy.

Gene therapy may be carried out according to generally accepted methods, for example, as described by Friedman, 1991. A virus or plasmid vector (see further details below), containing a copy of the gene encoding the ribozyme or antisense molecule linked to expression control elements and capable of replicating inside the target cells, is prepared. Suitable vectors are known, such as disclosed in US Patent 5,252,479 and International Patent Application Publication Number WO 93/07282 (Boehringer Ingelheim International GmbH). The vector is then injected into the patient, either locally at the site of the target or systemically. If the transfected gene is not permanently incorporated into the genome of each of the targeted cells, the treatment may have to be repeated periodically.

Gene transfer systems known in the art may be useful in the practice of the gene therapy methods of the present invention. These include viral and nonviral transfer methods. A number of viruses have been used as gene transfer vectors, including papovaviruses, eg SV40 (Madzak *et al,* 1992), adenovirus (Berkner, 1992; Berkner *et al,* 1988; Gorziglia and Kapikian, 1992; Quantin *et al,* 1992; Rosenfeld *et al,* 1992; Wilkinson *et al,* 1992; Stratford-Perricaudet *et al,* 1990), vaccinia virus (Moss, 1992), adeno-associated virus (Muzyczka, 1992; Ohi *et al,* 1990), herpesviruses including HS V and EBV (Margolskee, 1992; Johnson *et al,* 1992; Fink *et al,* 1992; Breakfield and Geller, 1987; Freese *et al*, 1990), and retroviruses of avian (Brandyopadhyay and Temin, 1984; Petropoulos *et al.,* 1992), murine (Miller, 1992: Miller *et al,* 1985; Sorge *et al,* 1984; Mann and Baltimore, 1985; Miller *et al,* 1988), and human origin (Shimada *et al,* 1991; Helseth *et al,* 1990; Page *et al,* 1990; Buchschacher and Panganiban, 1992). To date most human gene therapy protocols have been based on disabled murine retroviruses. Lentivirus-based vectors are preferred.

Nonviral gene transfer methods known in the art include chemical techniques such as calcium phosphate coprecipitation (Graham and van der Eb, 1973; Pellicer *et al,* 1980); mechanical techniques, for example microinjection (Anderson *et al,* 1980; Gordon *et al,* 1980; Brinster *et al,* 1981; Constantini and Lacy, 1981); membrane fusion-mediated transfer *via* liposomes (Felgner *et al,* 1987; Wancy and Huang, 1989; Kaneda *et al,* 1989; Stewart *et al,* 1992; Nabel *et al,* 1990; Lim *et al,* 1992): and direct DNA uptake and receptor-mediated DNA transfer (Wolff *et al,* 1990; Wu *et al,* 1991; Zenke *et al,* 1990: Wu *et al,* 1989b; Wolff *et al,* 1991; Wagner *et al,* 1990: Wagner *et al,* 1991: Cotten *et al,* 1990; Curiel *et al.* 1991a; Curiel *et al,* 1991b). Viral-mediated gene transfer can be combined with direct *in vivo* gene transfer using liposome delivery, allowing one to direct the viral vectors to the tumour cells and not into the surrounding nondividing cells. Alternatively, the retroviral vector producer cell line can be injected into tumours (Culver *et al*, 1992). Injection of producer cells would then provide a continuous source of vector particles. This technique has been approved for use in humans with inoperable brain tumours.

Other suitable systems include the retroviral-adenoviral hybrid system described by Feng et al (1997) Nature Biotechnology 15, 866-870, or viral systems with targeting ligands such as suitable single chain Fv fragments.

In an approach which combines biological and physical gene transfer methods, plasmid DNA of any size is combined with a polylysine-conjugated antibody specific to the adenovirus hexon protein, and the resulting complex is bound to an adenovirus vector. The trimolecular complex is then used to infect cells. The adenovirus vector permits efficient binding, internalization, and degradation of the endosome before the coupled DNA is damaged.

Liposome/DNA complexes have been shown to be capable of mediating direct *in vivo* gene transfer. While in standard liposome preparations the gene transfer process is nonspecific, localized *in vivo* uptake and expression have been reported in tumour deposits, for example, following direct *in situ* administration (Nabel, 1992).

Gene transfer techniques which target DNA directly to macrophages is preferred. Receptor-mediated gene transfer, for example, is accomplished by the conjugation of DNA (usually in the form of covalently closed supercoiled plasmid) to a protein ligand *via* polylysine. Ligands are chosen on the basis of the presence of the corresponding ligand receptors on the cell surface of the target cell/tissue type. The mannose receptor is rather specific for macrophages and specific targeting of DNA/mannose conjugates has been demonstrated. These ligand-DNA conjugates can be injected directly into the blood if desired and are directed to the target tissue where receptor binding and internalization of the DNA-protein complex occurs. To overcome the problem of intracellular destruction of DNA, coinfection with adenovirus can be included to disrupt endosome function.

The compound is one which inhibits the activity of PPARδ protein activity.

Suitable compounds may be selected using some of the methods described below.

It will be appreciated that the effective amount of an inhibitor of PPARδ activity may be administered to the patient in any suitable way and in any suitable formulation. Suitability of any method of administration or of any formulation may readily be determined by the skilled person by reference to the nature of the inhibitor. For example nucleic acid-based inhibitors may be formulated and administered in different ways to small molecule-based inhibitors as is known in the art.

The aforementioned compounds of the invention or compounds for use in the methods of the invention or a formulation thereof may be administered by any conventional method including oral and parenteral (eg subcutaneous, intravenous, or intramuscular) injection. The treatment may consist of a single dose or a plurality of doses over a period of time.

Typical doses are expected to be in the range 0.01-20mg/kg.

Whilst it is possible for a compound of the invention to be administered alone, it is preferable to present it as a pharmaceutical formulation, together with one or more acceptable carriers. The carrier(s) must be "acceptable" in the sense of being compatible with the compound of the invention and not deleterious to the recipients thereof. Typically, the carriers will be water or saline which will be sterile and pyrogen free.

The formulations may conveniently be presented in unit dosage form and may be prepared by any of the methods well known in the art of pharmacy. Such methods include the step of bringing into association the active ingredient (compound of the invention or compound for use in the methods of the invention) with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations in accordance with the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (eg povidone, gelatin, hydroxypropylmethyl cellulose), lubricant, inert diluent, preservative, disintegrant (eg sodium starch glycolate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide desired release profile.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The formulations mentioned herein are carried out using standard methods such as those described or referred to in reference texts such as the British and US Pharmacopoeias, Remington's Pharmaceutical Sciences (Mack Publishing Co.), Martindale The Extra Pharmacopoeia (London, The Pharmaceutical Press).

The therapeutic methods of the invention may be used to treat any mammal including humans. Thus, it is envisaged that the method may be used to treat, by way of example, domestic animals such as cats and dogs, or farm animals such as horses, cows, sheep, goats and pigs, or other commercially important mammals. When the inhibitor of PPARδ activity is a nucleic acid-based molecule it will be appreciated that it will be designed by reference to the mammal's PPARδ gene or cDNA.

It is preferred that the patient to be treated is a human patient.

A further aspect of the invention provides an inhibitor of PPARδ activity as claimed for use in medicine.

The inhibitor is packaged and presented for use in medicine. In particular, it may be packaged and presented for use in treating atherosclerosis, or in treating heart disease or treating stroke or treating peripheral artery disease or treating vascular disease associated with plaque formation or treating thrombotic blockage of blood vessels or treating Alzheimer's disease or treating cancer.

- A still further aspect of the invention provides a pharmaceutical composition comprising an inhibitor of PPARδ activity as claimed and a pharmaceutically-acceptable carrier.

Yet still further aspects of the invention provide use of an inhibitor of PPARδ activity as claimed in the manufacture of a medicament for preventing or reducing foam cell development, from macrophages or other cells, or removing foam cells, in a patient; use of an inhibitor of PPARδ activity as claimed in the manufacture of a medicament for preventing or treating a vascular disease associated with plaque formation and/or thrombotic blockage of the blood vessels; use as claimed of an inhibitor of PPARδ activity in the manufacture of a medicament for treating or preventing cancer.

Suitably, the macrophages or other cells are those involved in atherosclerotic pathology.

Until the present work, it was not envisaged that inhibitors of PPARδ activity would be useful, particularly in medicine. Also described are methods of identifying compounds which inhibit PPARδ activity and, in particular, which inhibit PPARδ activity in macrophage cells or in foam cells. The compounds so-identified may themselves be useful in medicine or they may be useful as lead compounds for further development into medically useful compounds. These methods described, or means for carrying out such methods, may be considered to be "drug screening methods" or "drug screening assays".

The application describes a method of identifying a compound which may, or which can be used as a lead compound to produce a compound which may, be useful in medicine the method comprising selecting a compound which inhibits PPARδ activity. Typically, the method may involve the screening of a number of test compounds which are present in a library of test compounds. Thus. identifying a compound which inhibits PPARδ activity comprises selecting from a library of test compounds any compound which inhibits PPARδ activity.

The above-mentioned methods may be used to select compounds which inhibit PPARδ gene expression or the production of PPARδ protein (ie translation) in a cell or which inhibit the PPARδ protein activity.

Suitable assay formats are well known to the person skilled in the art, and some of them are described in more detail in Example 3.

In relation to identifying compounds which inhibit PPARδ gene expression the PPARδ gene promoter operatively linked to a reporter gene such as, for example, luciferase. β-galactosidase, alkaline phosphatase or the like can be used. The expression of the reporter gene in a cell may be measured in the presence or absence of a test compound. Test compounds which selectively reduce the expression of the reporter gene are selected as compounds which may be useful in inhibiting or reducing expression of the PPARδ gene so that there is inhibition of PPARδ activity.

In relation to compounds which inhibit PPARδ protein activity, one particular activity which it is desirable to inhibit is the ability of the PPARδ protein to transcriptionally activate genes under its control. Thus, a reporter gene, such as one of those listed above, is operatively linked to a promoter or enhancer sequence which is under the control of PPARδ. Genes which are under the control of PPARδ include CYP4A6, acyl CoA oxidase and lipoprotein lipase; thus, the promoters of these genes are believed to be useful in this embodiment of the invention. Synthetic enhancer elements derived from these genes, linked to heterologous promoters such as from the HSV thymidine kinase gene, may also be useful in this embodiment. A genetic construct containing the promoter or enhancer/reporter gene is transfected into a suitable cell such as a monocyte or macrophage cells. COS-1 or CV-1 cells, both sublines of African Green Monkey kidney cells, may also be used. A genetic construct encoding PPARδ is also transfected into the cell and the ability of a test compound to inhibit the expression of the reporter gene is assessed. The transfected cells would be incubated with a known PPARδ agonist such as Compound F as disclosed on page 9 of WO 97/28149 (hereinafter referred to as Compound F) in combination with the test compound and the ability of the test compound to interfere with the action of the agonist would be determined. Compounds which reduce or inhibit the expression of the reporter gene are selected for further study.

A CARLA (co-activator/receptor/ligand interaction assay) may be used as described in Example 3. These assays rely on the dependence of the binding of co-receptors on the presence of a suitable ligand for PPARδ. In the CARLA assays, an agonist is present and the ability of the test compound to interfere with the action of the agonist is assessed.

Particularly preferred compounds which may be selected are those which bind to the ligand binding portion of PPARδ in a transcriptionally futile manner and thereby prevent the action of agonistic ligands of PPARδ.

It is preferred if a "pre-screening" step is carried out to identify compounds bind to PPARδ before determining whether they are an inhibitor or antagonist of PPARδ activity. Receptor-ligand binding assays are well known in the art and can readily be devised and executed by the suitably skilled person.

A "pre-screening" step may be carried out which is essentially the foam cell assay described below. Thus, in this way compounds may be selected which are active in preventing or reducing foam cell development from macrophages or other cells or remove foam cells, and which are inhibitors of PPARδ.

Suitably, the macrophages or other cells are those involved in atherosclerotic pathology.

PPARδ inhibitors may be identified using a two-hybrid approach to monitor the interaction of PPARδ and co-activators such as SRC-1. In this case, PPARA (or a functional portion thereof) is fused to a DNA binding protein (such as the DNA binding domain of GAL4) and the co-activator molecule or a functional portion thereof (such as SRC-1) would be fused to a transactivating protein (such as the transactivation domain of VP16). Yeast expressing both of these fusions and having an integrated reporter gene (such as β-galactosidase) under the control of a DNA binding protein-dependent (eg GAL4-dependent) promoter are generated.

The yeast are grown in the presence of a PPARδ agonist and a wide range of test compounds. Cultures with low β-galactosidase activity indicate the presence of a PPARδ antagonist.

It will be appreciated that the methods of identifying a compound as described above are useful in identifying compounds which may, or which can be used as a lead compound to produce a compound which may, (1) prevent or reduce foam cell development from macrophages or remove foam cells, (2) prevent or be useful in treating a vascular disease associated with plaque formation and/or thrombotic blockage of the blood vessels, (3) prevent or be useful in treating cancer, or (4) prevent or be useful in treating Alzheimer's disease.

Once a compound has been selected for its ability to inhibit or reduce PPARδ activity, it may conveniently be used in a further screen to determine, for example, whether it may prevent or reduce foam cell development from macrophages or other cells, or remove foam cells, whether it may prevent or is useful in treating a vascular disease associated with plaque formation and/or thrombotic blockage of the blood vessels, or prevent or is useful in treating cancer, or prevent or is useful in treating Alzheimer's disease.

Suitably, the macrophages or other cells are those involved in atherosclerotic pathology.

Screening methods to determine these properties are known from this application (in relation to foam cell development from macrophages) or are known in the art.

Other, further, useful screens may be carried out on the selected compound to determine, for example, its solubility, its pharmacological profile and drug metabolism profile. Similarly, screens may be carried out to determine the compounds selectivity for inhibiting PPARδ activity. For example, screens may be carried out to determine whether a particular compound may modify the activity of another PPAR isoform. Typically, it is preferred if compounds are selected which substantially do not modify the activity of other PPAR isoforms. However, it may be advantageous if the compound which is selected is one which inhibits PPARδ activity but is an agonist or activator of PPARγ activity. Equally, it may be advantageous if the compound which is selected is one which inhibits PPARδ activity but is an activator or agonist of PPARα activity.

The compound selected in the screening methods of the invention may be a "drug-like compound".

The term "drug-like compound" is well known to those skilled in the art, and may include the meaning of a compound that has characteristics that may make it suitable for use in medicine, for example as the active ingredient in a medicament. Thus, for example, a drug-like compound may be a molecule that may be synthesised by the techniques of organic chemistry, less preferably by techniques of molecular biology or biochemistry, and is preferably a small molecule, which may be of less than 5000 daltons molecular weight and which may be water-soluble. A drug-like compound may additionally exhibit features of selective interaction with a particular protein or proteins and be bioavailable and/or able to penetrate target cellular membranes, but it will be appreciated that these features are not essential.

The term "lead compound" is similarly well known to those skilled in the art, and may include the meaning that the compound, whilst not itself suitable for use as a drug (for example because it is only weakly potent against its intended target, non-selective in its action, unstable, poorly soluble, difficult to synthesise or has poor bioavailability) may provide a starting-point for the design of other compounds that may have more desirable characteristics.

The terms "drug-like compound" and "lead compound" typically refer to relatively small organic molecules rather than, for example, relatively large nucleic acid-based molecules. Nevertheless, it will be appreciated that many embodiments of the screening methods of the invention are suitable for identifying nucleic acid-based molecules, particularly those which inhibit expression of the PPARδ gene or translation of the PPARδ protein.

As is well known in the art of drug discovery, a lead compound found in a screen may be modified and the modified compound screened to determine its activity.

The application also describes an *in vitro* model for dietary fat-induced foam cell formation and its use in identifying useful compounds.

Foam cell formation is in the first step a two step process wherein the cell is converted into a foam cell precursor by being subjected to an inflammatory stimulus such as PMA or by being brought into contact with a suitable growth factor such as GMCSF (Granulocyte macrophage colony stimulating factor). In the second step the precursor cells become foam cells in the presence of a suitable concentration of fatty acids.

Thus, application describeds a method of identifying a compound which may, or which can be used as a lead compound to produce a compound which may, (1) prevent or reduce foam cell development from macrophages or other cells, suitably those macrphages or other cells involved in atherosclerotic pathology, or remove foam cells or (2) prevent or be useful in treating a vascular disease associated with plaque formation and/or thrombotic blockage of the blood vessels, the method comprising the steps of selecting:
(a) a suitable primary cell population or cell line,
(b) supplying to the cell line an inflammatory stimulus or growth factor which facilitates differentiation to a foam cell precursor,
(c) supplying to the foam cell precursor a suitable concentration of fatty acid, and
(d) measuring the accumulation of fatty acid in the cell in the presence or absence of the compound.

A suitable primary cell population or cell line is that involved in atherosclerotic pathology.

The cell type may be any suitable cell type. Preferably, it is a leukocyte cell. It is particularly preferred that the leukocyte cell line is a macrophage or macrophage precursor cell line.

Typically, the cells are THP-1 or U937. THP-1 is available as ECACC cell deposit No. 85011440, and U937 is available as ECACC cell deposit No. 88081201. However, any suitable leukocyte cell may be used, although it is preferred if a monocytic or macrophage-like cell is used. THP-1 cells are "monocyte-like" and can be differentiated to macrophage-like cells by phorbol ester (PMA).

By "supplying an inflammatory stimulus to the cell" we include the supply of any suitable inflammatory stimulus which facilitates stimulation of the cells into foam cells.

By "growth factor which facilitates the differentiation to foam cells" we include any suitable growth factor such as GMCSF and, in the context of the invention, PMA. PMA or a functionally equivalent phorbol ester is preferred.

Typically, the phorbol ester is PMA but it may be any suitable phorbol ester which facilitates the differentiation of the cells.

Typically, PMA is used at a concentration of between 0.1 to 10ng/ml.

The fatty acid may be supplied to the cell in the serum in which the cell is growing. We have found that heat inactivated fetal calf serum from Gibco BRL gives a low background of lipid accumulation, whereas a high level of lipid accumulation is achieved using Controlled Process Serum Replacement (CPSR-3) from Sigma. Alternatively, it may be supplied (for example by adding to the growth culture medium) in a suitable concentration. Suitable fatty acids include mono- and polyunsaturated fatty acids of chain lengths 18-22, including linoleic acid. Suitably, the fatty acids are supplied at a concentration of between 10 to 100µM.

Accumulation of fatty acid in the cell may be measured using any suitable method. Particularly convenient methods involve the use of fatty acid stains such as Nile Red or Oil Red O which can be used *in situ* and read with a plate reader. Other methods which can be used to measure accumulation of fatty acids include TLC, HPLC, and LC-MS.

The accumulation of fatty acid in the cell in the presence or absence of test compound is assessed and compounds which lead to a reduction in the accumulation of fatty acid in the cell are selected for further testing.

It will readily be appreciated that since the cells can be grown in multi-celi culture dishes (eg 64 cell culture dishes) and since the lipid staining can be quantitated, for example optically in a microtitre plate reader, the screening method may readily be automated for high throughput screening.

The compounds identified and identifiable by the screening methods described herein are believed to be useful in for treatments. The invention will now be described in more detail by reference to the following Description, Examples and Figures wherein:

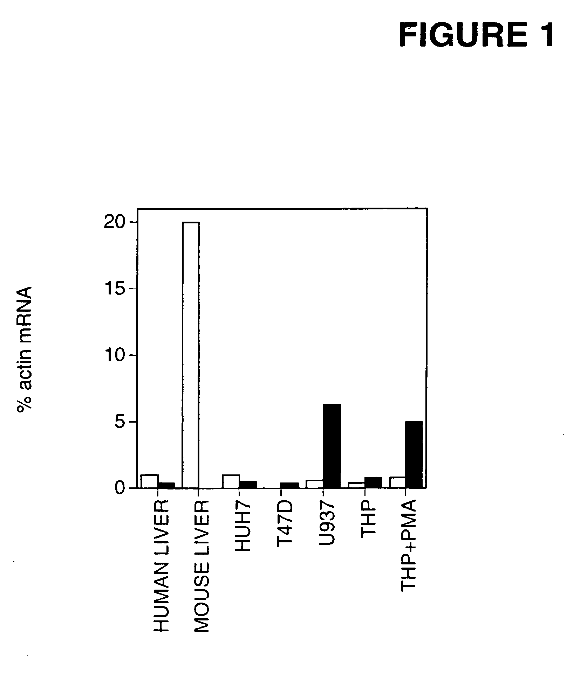

**Figure 1** shows the expression of PPARs in THP-1 cells. RNAase expression analysis for PPARα and δ mRNAs was performed on total RNA from mouse and human liver and several human cell lines. The Huh7 cell line is a human liver cell line that contains identical amounts of PPARδ (black bars) and α (white bars) mRNA when compared to total RNA isolated from human liver (>1% of actin). Both Huh7 and human liver do not respond to peroxisome proliferators in terms of increases in lipid metabolism and transcription from PPRE containing reporter constructs. Monocytic cell lines such as U937 and THP-1 cells express relatively high levels of PPARδ (5-6% of actin). Other human tissues were analysed for α and δ expression such as colon, ovary, testes, kidney, adrenal. In all cases the expression levels were less than 1% of actin (data not shown). However, it is possible that individual cell components of these complex tissues express significant levels of these receptors.

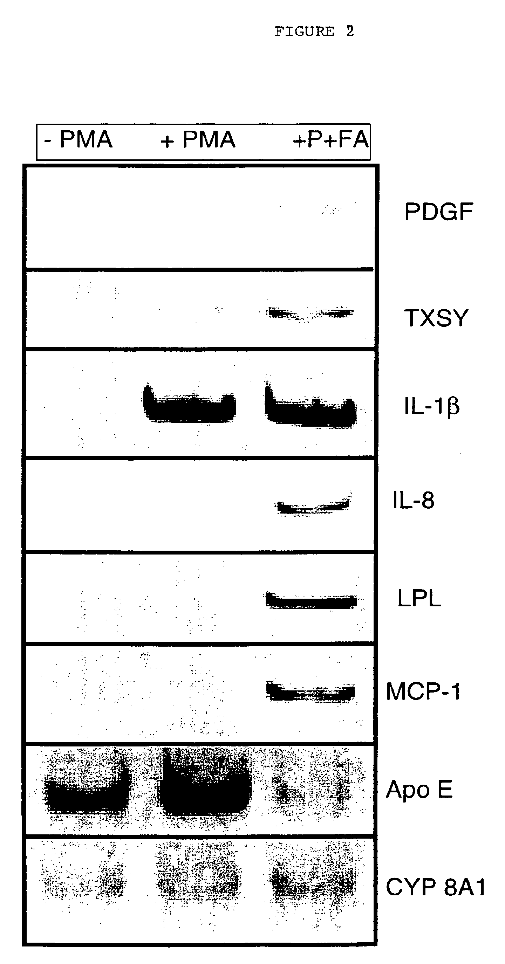

**Figure 2** shows atherogenic gene expression in THP-1 cells. Total RNA from THP-1 cells was analysed by RT-PCR for the expression of various gene products associated with the atherogenic process. Shown are ethidium bromide-stained agarose gels of the analysis of THP-1 cells in the normal growing conditions (-PMA), adherent THP-1 cells after phorbol ester treatment (+PMA) and phorbol ester treatment in the presence of 100µM linoleic acid. All probes were designed to cross intron/exon boundaries and did not amplify genomic DNA.

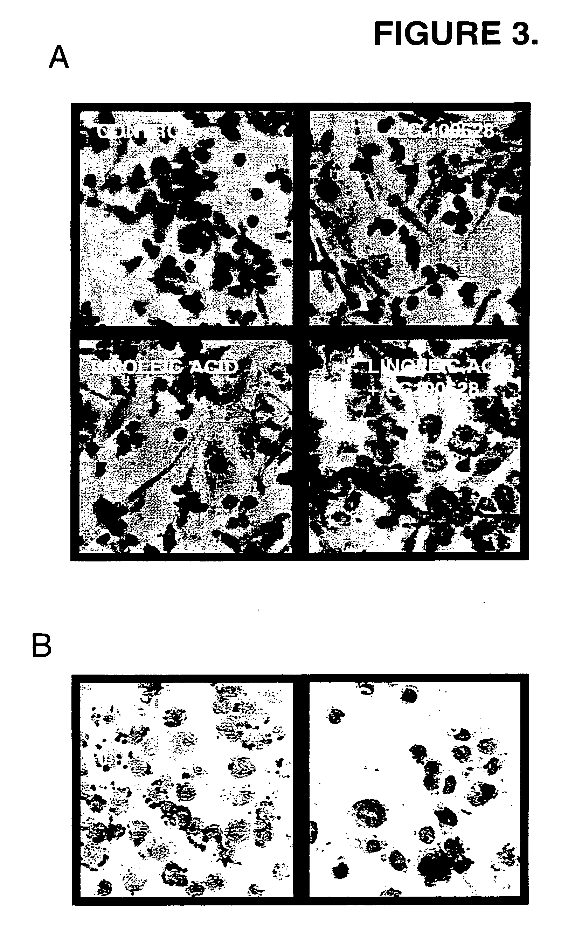

**Figure 3** shows that fatty acids and rexinoids promote the formation of foam cells that are inhibited by rosiglitazone. Rexinoids are drugs specific for RXR. such as 9-cis-retinoic acid and the Ligand Pharmaceutical compound. LG100268 (Mukherjee R et al., 1997 Nature 386, 407-410). Rosiglitazone is made by SmithKline Beecham and is also known as *Avandia* and BRL49653.

### A. Fatty acid co-operate with rexinoids in foam cell formation.

THP-1 cells were treated with 5ng/ml PMA and DMSO (dimethyl suiphoxide. 0.5%), linoleic acid (30 µM), LG 100268 (5 µM) and linoleic acid with LG 100268. Treatment was continued for 3 days and the cells fixed in 0.66M paraformaldehyde, stained with a saturating solution of Oil Red O and then counterstained with haematoxylin. Increased lipid accumulation is observed with treatment with either linoleic acid or LG 100268. Addition of both compounds produces an additive effect on lipid accumulation.

### B. Rosiglitazone promotes lipid clearance.

PMA treated THP-1 cells were treated with 20µM BRL49653 for 3 days and stained for lipid accumulation as described above.

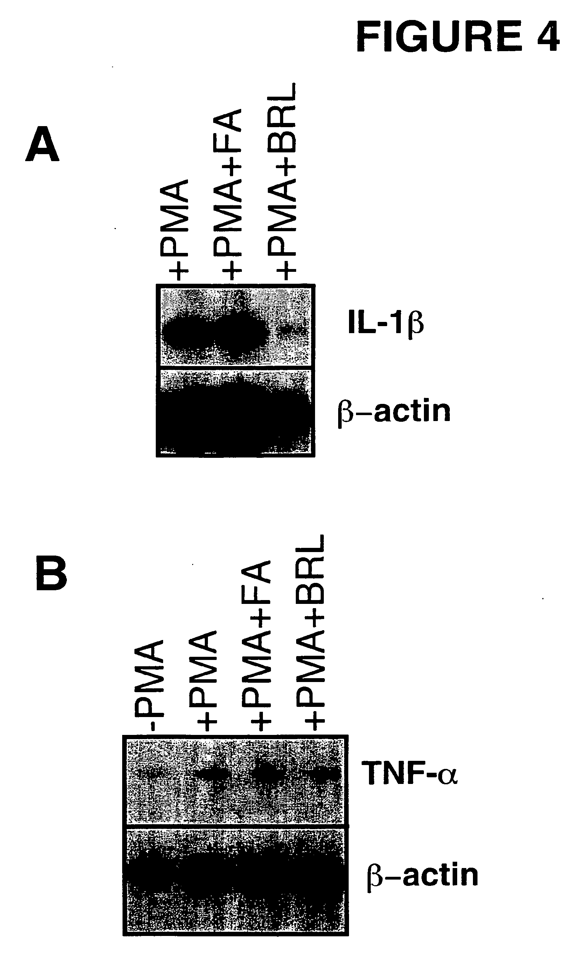

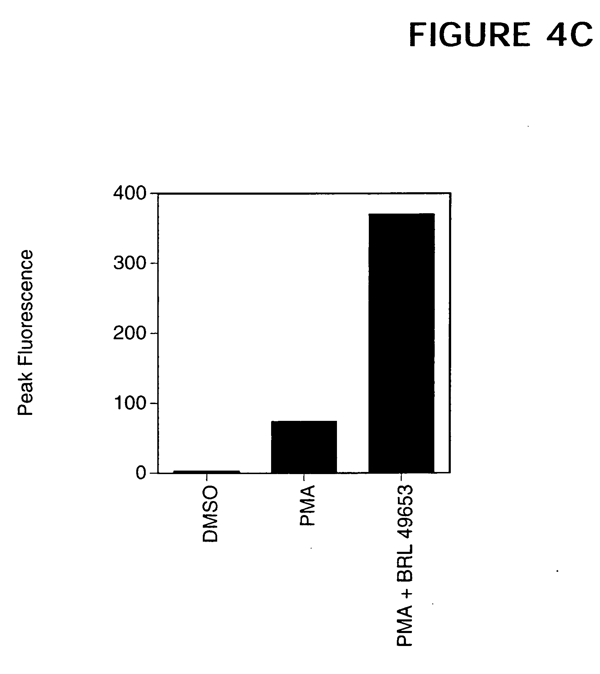

**Figure 4** shows that rosiglitazone modulates gene expression in THP-1 cells.

### A. IL-1β expression is reduced by rosiglitazone.

RNase protection analysis of interleukin 1β mRNA levels in PMA treated THP-1 cells (+PMA), PMA and linoleic acid treated cells (+PMA+FA), and PMA and BRL49653 treated cells (+PMA+BRL). Also shown is the actin normalisation signal.

### B. TNFα expression is reduced by rosiglitazone.

RNase protection analysis of TNFα mRNA levels in control THP-1 cells (-PMA), PMA treated cells (+PMA), PMA and linoleic acid treated cells (+PMA+FA), and PMA and BRL49653 treated cells (+PMA+BRL). Also shown is the actin normalisation signal.

### C. CD36 expression is increased by rosiglitazone.

Fluorescence Activated Cell Sorting (FACS) analysis of CD36 antigen expression in control THP-1 cells (-PMA), and PMA and BRL49653 treated cells (+PMA+BRL). Treatment was for 48 hours at 50µM BRL49653. Cells were scraped from the culture plates, fixed and incubated with a FITC (fluorescein isothiocyanate) conjugated CD36 antibody (Coulter Immunotech). FACS analysis was performed using a Coulter EPIC V cell sorter.

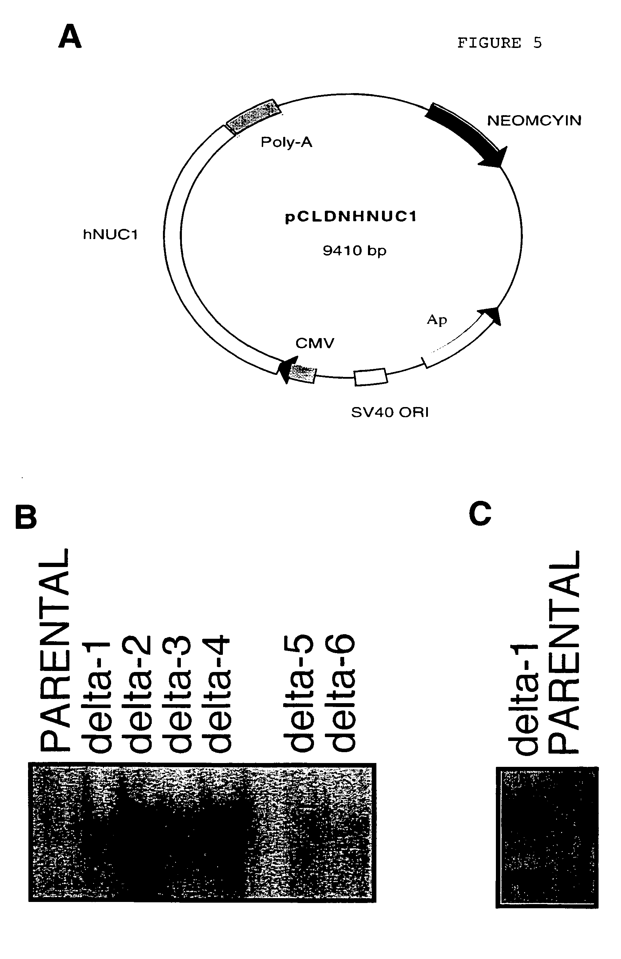

**Figure 5** shows the generation of cell lines constitutively expressing PPARδ. The entire coding sequence of human PPARδ was subcloned into the eukaryotic expression vector pCLDN (Brighty D W et al Proc. Natl. Acad. Sci. USA 88, 7802-7805 (1991)). The resulting plasmids were transfected into THP-1 cells using a modified DEAE-Dextran procedure (Fujita et al (1986) Cell 46, 401-407). The cells were maintained in medium containing 1mg/ml G418 and 10% THP-1 conditioned medium, with rigorous washing procedures to remove dead cells. This was continued until cell killing stopped and robust growth was observed. We obtained 6 lines each of pCLDN (empty vector) pCLDNPPARδ, pCLDNPPARδAS, pCLDNPPARα and pCLDNPPARαAS. "AS" means antisense construct. All of these cell lines were essentially identical in growth and viability. Initial differentiation experiments were performed with the PPARδ cell lines in the selection medium. These were negative for all constructs including the empty vector control line. The cells for all constructs were still alive after PMA treatment apart from the PPARδ anti-sense lines which all showed trypan blue uptake. The cells were then cultured without G418 until the empty vector control lines responded to PMA in a similar fashion to the parental control. This then revealed that the pCLDNPPARδ lines all differentiated in a similar fashion but the pCLDNPPARδAS lines all failed to differentiate.

RNA and protein was prepared from the PPARδ sense cell lines and analysed for PPARδ mRNA by RNase protection (B) and for PPARδ protein by western blotting (C). All 6 lines expressed >10 fold increases in PPARδ mRNA over the parental cell line. These levels are far in excess of those observed in PMA treated cells. High levels of PPARδ protein have been seen in lines designated delta-1 and delta-2. The other 4 lines have not been analysed by western blotting. All subsequent experiments were performed on these lines.

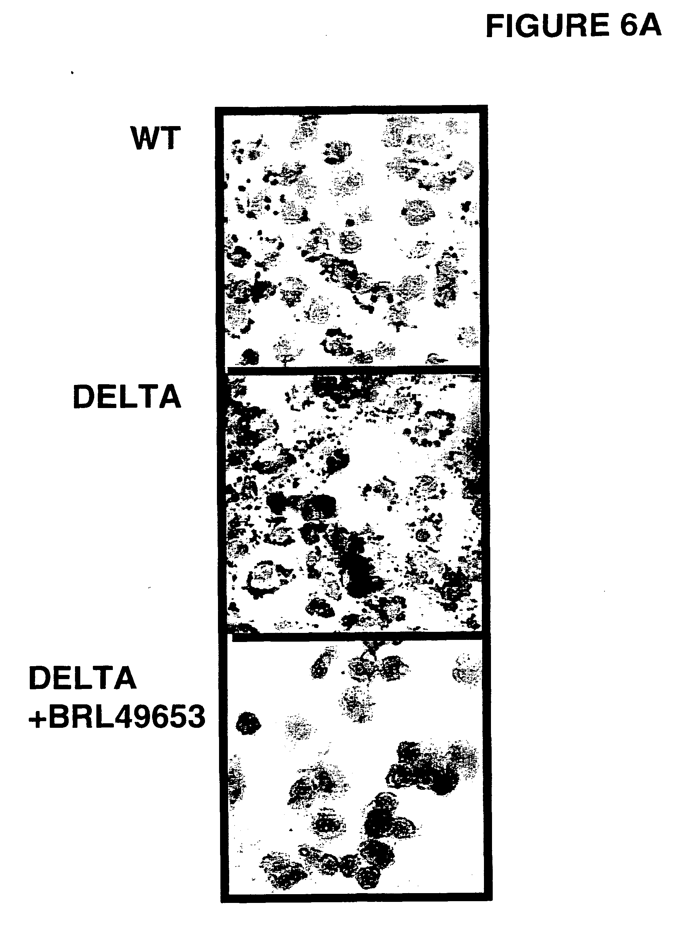

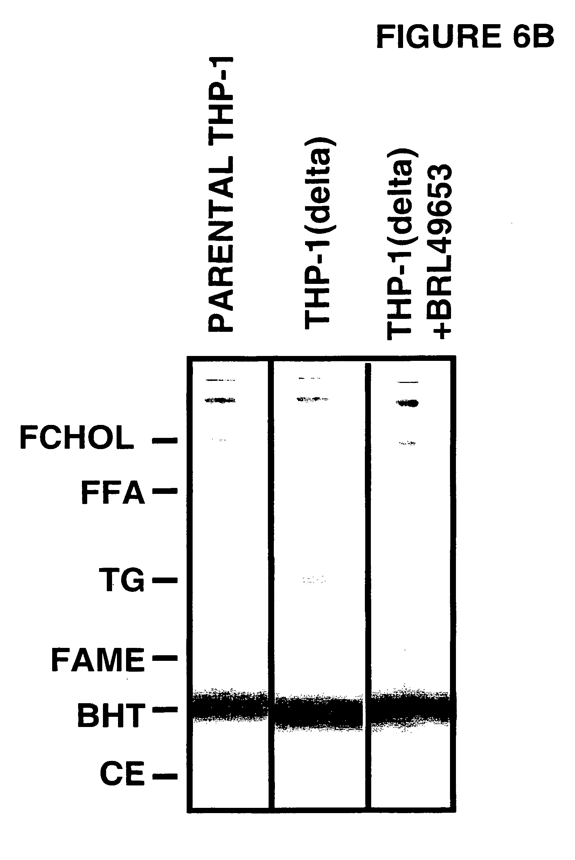

**Figure 6** shows that PPARδ over-expression promotes foam cell formation.
A. Parental THP-1 cells and pCLDNPPARδ cells were treated with 5ng/ml PMA and DMSO (0.5%) or BRL49653 (50µM). Treatment was continued for 3 days and the cells fixed in 0.66M paraformaldehyde, stained with a saturating solution of Oil Red O and then counterstained with haematoxylin. Subsequent experiments have shown BRL49653 action to be effective at submicromolar concentrations.
B. Cultures were treated as above and then extracted with methanol/chloroform/PBS (1:1:1 vol/vol). The organic phase was run on a silica gel TLC plate (Merck:Kieselgel 60 with concentration zone) with heptane (80%), diethyl ether (18%) and acetic acid (2%) as the solvent. The lipids were then stained with an aerosol of phosphomolybdic acid in methanol. Shown is the relative mobility of free cholesterol (FCHOL), free arachidonic acid (FFA), highly unsaturated triacylglycerol (TG), fatty acid methyl esters (FAME), cholesterol esters (CE) and also butylated hydroxyanisole (BHT) that is present in the solvent system.

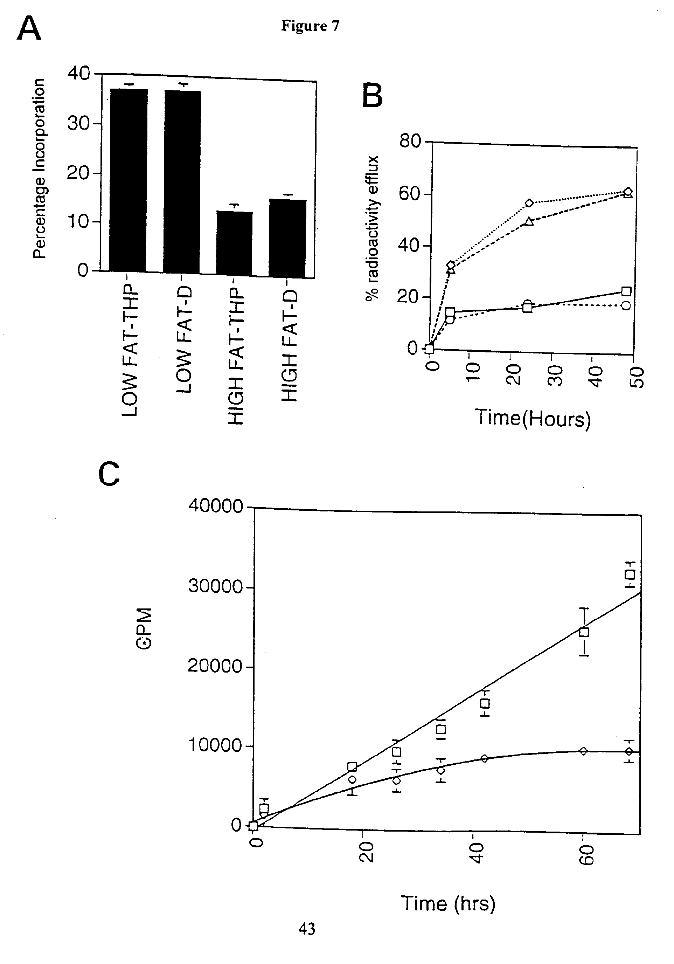

**Figure 7** shows that fatty acid efflux is repressed in activated PPARδ over-expressing cells.

### A. Fatty acid uptake.

1 million cells in 2ml of medium were incubated with 5 µCi of ³H-oleic acid overnight with or without 100µM linoleic acid (HIGH FAT and LOW FAT respectively). The cells were then pelleted, washed and resuspended in fresh medium without labelled fatty acids. The percentage incorporation was determined by scintillation counting of an aliquot of the medium and the washed cells. All assays were performed in triplicate.

### B. Fatty acid efflux.

The washed cells were incubated for a further 50 hours and the release of radioactivity was monitored over time. Parental cells in high fat are shown as diamonds and in low fat are shown as squares. PPARδ over-expressing cells in high fat are shown as triangles and in low fat as circles.

### C. Fatty acid efflux of PMA activated cells.

Cells were treated with PMA and incubated with radiolabelled oleic acid as described above. The adherent cells were washed and the release of radioactivity from these cells was measured over time. The parental cell line (squares) displays higher levels of radioactivity release than the PPARδ over-expressing cells (diamonds).

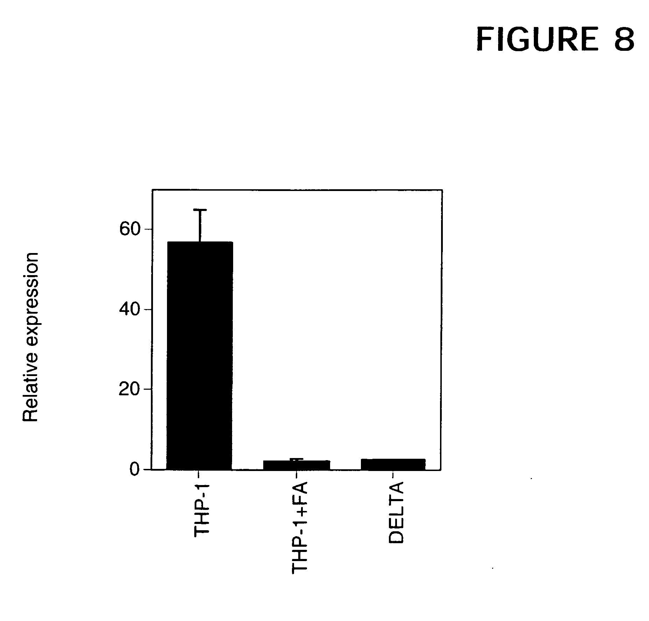

**Figure 8** shows that ApoE gene expression is regulated by fatty acids through PPARδ.

Total RNA was prepared from parental THP-1 cells treated with PMA (THP-1), or PMA and linoleic acid (THP-1+FA), and untreated PPARδ over-expressing cell line (DELTA). cDNA was prepared from these RNA samples and the cDNA was analysed for ApoE sequences using TAQMAN procedures. The result shown is relative expression normalised for β-actin expression and the error bars represent the standard deviation in duplicate samples.

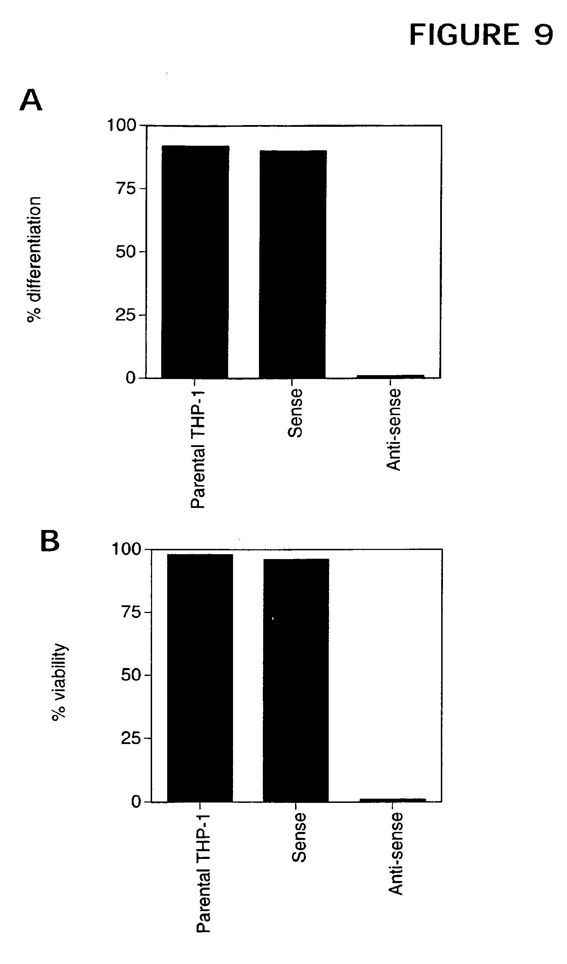

**Figure 9** shows that inhibition of PPARδ prevents foam cell formation. Cells were treated overnight with 5ng/ml PMA and scored for differentiation (adherence to culture dish) and viability (trypan blue exclusion). The parental THP-1 cells and PPARδ over-expressing cells (Sense) differentiate well in the presence of PMA. However, the PPARδ antisense expressing cells (Anti-sense) do not differentiate at all and become highly permeant to trypan blue. These PPARδ anti-sense cells grow normally with high viability in the absence of PMA.

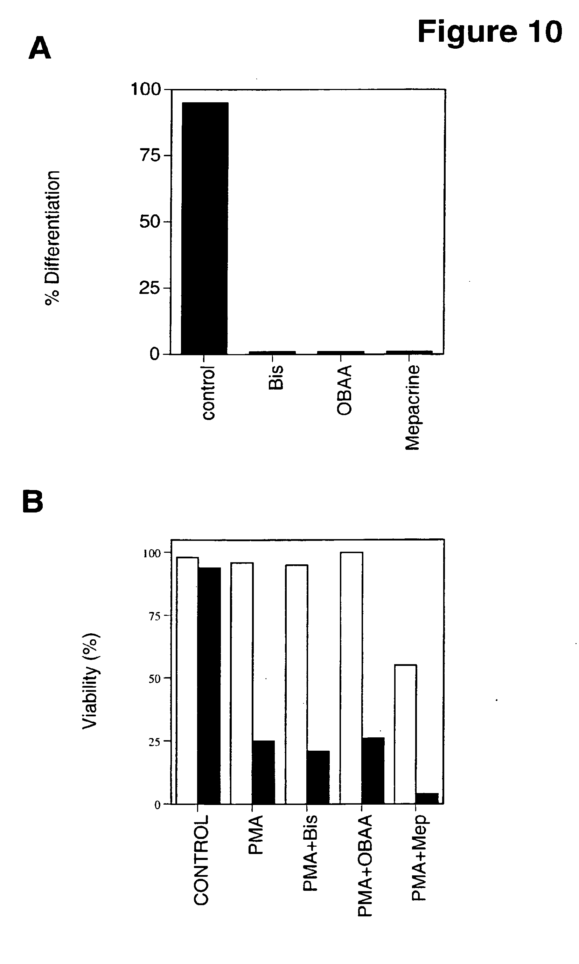

**Figure 10** shows that PPARδ protection from death is not downstream of PKC. Parental and PPARδ anti-sense cells were incubated with PMA and an inhibitor of PKC activity (Bis) and two inhibitors of PLA2 activity (OBAA and mepacrine). These inhibitors were included at concentrations used in previous studies for specific inhibition.

Bis is bisindoylmaleamide I and is available from Sigma. OBAA is 3-(4-octadecyl)benzoylacrylic acid and is available from Calbiochem. Mepacrine is 6-chloro-9-[(4-diethylamino)-1-methyl butyl]amino-2-methoxy-acridine and is available from Calbiochem.
All inhibitors prevented the differentiation of parental THP-1 cells (A). This confirmed the role of PKC in the action of phorbol ester. The action of the PLA2 inhibitors appears to reveal new aspects of the macrophage differentiation. However, when we examined the viability of these cells by trypan blue exclusion (B) we found that they did not block the PMA-induced "death" of the PPARδ anti-sense cell line (black bars). Only mepacrine appeared to have any sign of toxicity in the parental cells (white bars).

**Figure 11** shows that lipid accumulation is promoted by an agonist for PPARδ. Compound F.

**Figure 12** shows that Compound F, a PPARδ agonist, promotes macrophage foam cell formation from THP-1 glial cells.

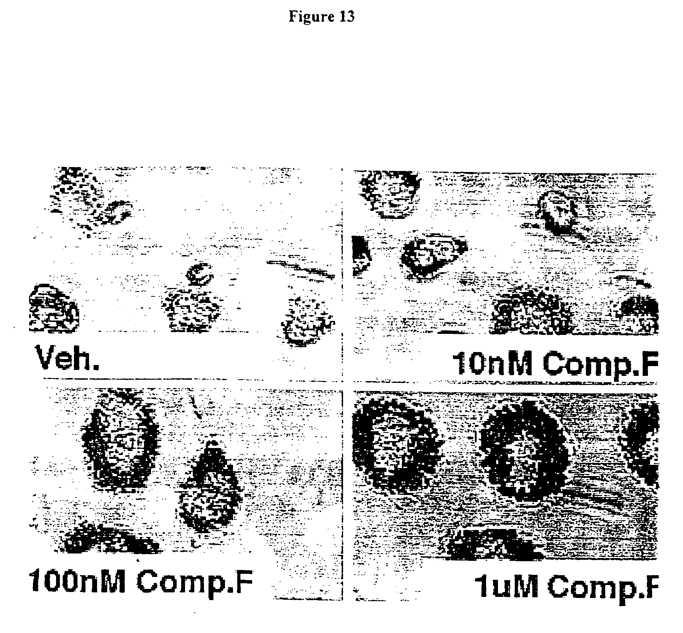

**Figure 13** shows that Compound F promotes macrophage foam cell formation from primary human monocytes.

**Figure 14** shows lipid accumulation in glial cells promoted by PEARδ.

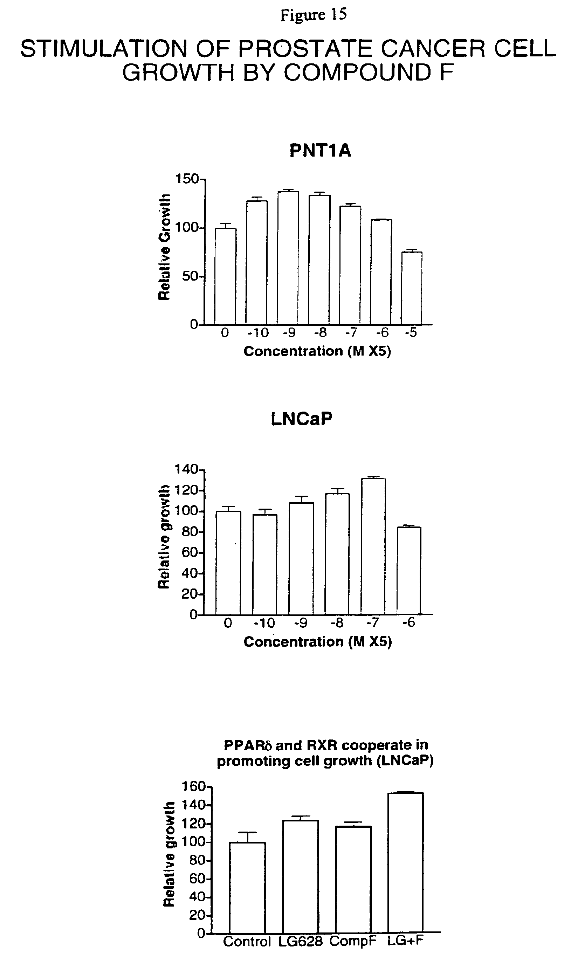

**Figure 15****:** shows that Compound F stimulates proliferation of prostate cancer cell lines.

### Description: The rôle of PPARδ in the formation of foam cells.

### Tissue culture

Human glioblastoma astrocytoma U373 cells were grown in RPMI 1640 culture medium supplemented with 10% v/v heat-inactivated foetal bovine serum (GibcoBRL, Paisley, Scotland), penicillin (5000IU/ml) (GibcoBRL, Paisley. Scotland) and streptomycin (5000µg/ml) (Gibco, Paisley, Scotland). The cultures were grown at 37°C and 5% CO₂ and passaged when 100% confuency was achieved.

### Drug treatment of the cell lines

Confluent cells were plated in six-well test plates in 2ml of the medium used for the tissue culture detailed above and treated with 5µM and 1µM of the PPARδ-specific agonist Compound F. DMSO was used as a control. The Compound and medium were renewed every three days and the cells treated for one week.

### Staining experiments

Oil Red O staining with haematoxylin counterstaining experiments were performed on cells from each of the treatments described in the paragraph above. The medium was discarded and the wells washed with PBS (2ml). The cells were fixed in 10% formalin (1ml) for one hour and then washed twice in PBS before brief rinsing in 60% iso-propyl alcohol. Oil Red O solution (1ml) (Sigma) was added and removed after three hours. The wells were then washed twice with PBS. For the counterstaining haematoxylin counterstain (1:10, 1mL) (Sigma) was added and removed after five minutes by washing twice with PBS. The second wash was left in the wells.

### Results

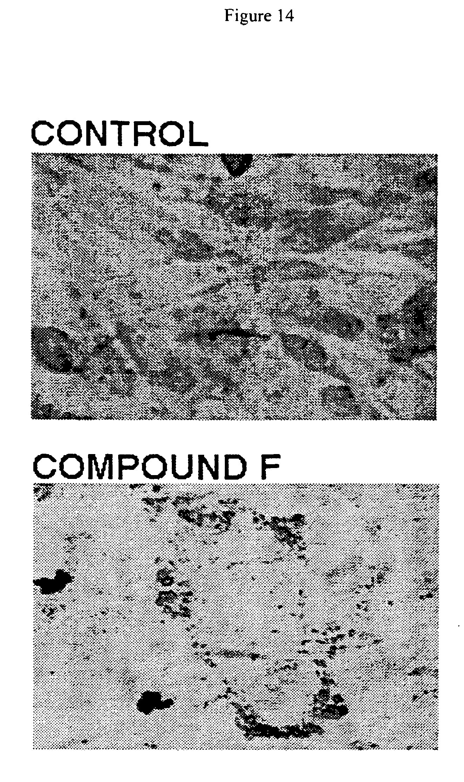

After one week, the control cells showed no lipid accumulation. For the cells treated with Compound F, lipid accumulation was observed and was notably macrovesicular i.e. lipid accumulation was present not only in the perinuclear area but also in the astrocytic processes (see **Figure 14**).

### Example 1: The role of PPARδ in the formation of atherosclerotic foam cells.

Activated macrophages express all three forms of peroxisome proliferator activated receptor, namely alpha (α), gamma (γ), and delta (δ). In the work described in Example 1 we demonstrate that expression of PPARδ is required for macrophage activation by phorbol esters and that PPARδ promotes macrophage foam cell formation. It has previously been shown that PPARγ agonists negatively regulate macrophage activation and have profound anti-inflammatory actions. In this Example we show that the PPARγ agonist, rosiglitazone, inhibits macrophage foam cell formation. Therefore, PPARγ and PPARδ mediate opposing roles in macrophage lipid metabolism and differentiation. These results indicate that, surprisingly, antagonistic ligands for PPARδ are strong candidates as drugs for the prevention of cardiovascular disease.
Experimental materials and methods are described in the Figure legends.

### Results

### Validation of foam cell model

Monocytic cell lines are widely used in basic research into the molecular basis of atherogenesis. We have investigated the influence of lipids and dietary fat, and the influence of stress and inflammation. The most versatile cell line for this research is the human cell line THP-1. This cell line grows in suspension under normal conditions. however inflammatory stimuli such as phorbol esters provoke a powerful differentiation and inflammatory response where the cells become adherent and more "macrophage like". This cell line is also known to respond to fatty acids and oxidised LDL by changes in gene expression, and oxidised LDL has been shown to promote cytoplasmic lipid accumulation. These regulatory phenomenon are viewed as representing the initial events that occur in atherosclerotic plaque formation (12).
We have now found an important link between the inflammatory process and the lipid pathology of these cells with the observation that phorbol ester stimulation of THP-1 cells leads to a large increase in the levels of PPARδ mRNA. Other groups have shown that inflammatory stimuli upregulate the expression of PPARγ in mouse and human macrophages, as well as monocyte derived cell lines such as THP-1 and U937 (13-15). PPARδ has also been shown to be expressed in thioglycolate-elicited peritoneal macrophages from mice.(13). We have also demonstrated that activated THP-1 cells express many inflammatory gene products associated with atherogenesis such as MCP (monocyte chemoattractant protein), IL-8, II-1β and thromboxane synthase (Figure 2), and have found that the expression of several of these gene products upon activation is modulated by fatty acids. The finding that the expression of apolipoprotein E increases upon activation and that this is severely repressed by fatty acids is significant. These results demonstrate that this system responds to fatty acids and inflammatory stimuli in a suitable manner for it to be considered a good model for human vascular disease. We also observed that treatment of adherent THP-1 cells with polyunsaturated fatty acids, such as linoleic acid, leads to the accumulation of lipid droplets in the cytoplasm (Figure 3A). These lipid laden macrophages are known as foam cells. The involvement of a PPAR/RXR heterodimer in the regulation of foam cell formation was suggested by the observation that an activating ligand for the retinoid X receptor (LG 100268) promoted foam cell formation in co-operation with fatty acids. Linoleic acid is not a very specific ligand for PPAR's, however it has been shown to activate PPAR alpha and delta efficiently, whereas it is a rather poor activator of PPARγ. Treatment of these cells with the PPARα activator Wy 14,643 had no effect, consistent with the low expression of this isoform in the activated macrophage (Figure 1). The use of rosiglitazone, BRL49653, also did not produce foam cells; however, it is known that these cells contain PPARγ. In fact BRL49653 treatment results in a reduction of lipid deposits when compared to the untreated cells (Figure 3A) and can inhibit the linoleic acid and rexinoid promotion of foam cells (data not shown). These data collectively suggest for the first time that PPARδ is the molecular target for fatty acids in the promotion of foam cell formation and that PPARγ is a negative regulator of foam cell function.

### Rosiglitazone (BRL49653) opposes foam cell formation.

Our data support the role of PPARγ in the antagonism of macrophage activation and lipid accumulation. We have also observed that cell adherence of THP-1 cells upon treatment with BRL49653 is slower than observed for the untreated cells (data not shown). This inhibitory role was also suggested by two papers in *Nature* in which the negative regulation of inflammatory mediators by PPARγ was demonstrated (13,14). We confirmed the anti-inflammatory action of TZD's by examining the effect of BRL49653 on IL-1β expression. Indeed BRL49653 treatment leads to a drop in IL-1β mRNA levels (Figure 4A). The reduction of TNFα mRNA, when compared to actin. was also observed, however this reduction was not so dramatic (Figure 4B). The uptake of lipid by the scavenger receptor CD36 has been shown to be regulated by PPARγ in THP-1 cells (16,17) and we find that CD36 is regulated by TZD in the activated THP-1 cell (Fig 3C), however these cells do not form foam cells (Fig 2). Therefore CD36 upregulation is not sufficient for foam cell formation and the overall effect of PPARγ activation is to antagonize foam cell formation. TZD's are the thiazolidinedione class of drugs which includes BRL 49653.

### Genetic modulation of foam cell model.

In order to ascertain whether or not PPARδ was involved in the fatty acid induced foam cell formation, cell lines were generated from THP-1 cells that over-expressed PPARδ or that expressed PPARδ anti-sense mRNA. The cDNA encoding PPARδ was cloned into a vector (pCLDN) that directs transcription under the control of a powerful enhancer from the human cytomegalovirus (Fig 5A). This vector also contains the neomycin resistance gene for the selection of targeted cells. The cDNA was inserted in both orientations in order to express the sense DNA for PPARδ protein expression (pCLDNPPARδ-S ) and in the anti-sense direction to block PPARδ expression (pCLDNPPARδ-AS). The THP-1 cells were transfected with pCLDNPPARδ-S or pCLDNPPARδ-AS and selected with 1mg/ml G418 until robust growth in G418 was observed. The cells were then released from G418 selection as even the vector alone-controls would not differentiate in the presence of G418. G418 is known to be an inhibitor of protein kinase C activity and therefore is an antagonist of phorbol ester action. After recovery from G418 selection. over-expression of PPARδ in six independently transfected polyclonal cell lines was confirmed by RNAase protection (Fig 5B) and western blotting (Fig 5C). These cell lines grow normally and do not display any adherence to the tissue culture flasks: however, when treated with PMA, they become adherent and accumulate large amounts of intracellular lipid when compared to the wild type cells, even in the absence of added fatty acids (Fig 6). BRL49653 treatment can prevent the PPARδ-driven foam cell formation. Fatty acid analysis demonstrated that the increases in cellular lipid are mainly in triglycerides and that this pool is eliminated by the action of BRL49653 (Fig 6B). Using tritiated oleic acid we have analysed the uptake and efflux of fatty acids in parental and PPARδ over-expressing cell lines. We have found that uptake and efflux are essentially identical between the two cell lines in the non-activated state (Fig 7A and B). In the activated cells, however, it is apparent that efflux is markedly lower from the PPARδ over-expressing cell lines (Fig 7C).

PPARδ over-expressing cells have ApoE mRNA levels that are dramatically reduced (35 fold) compared to the parental cell line (Fig 8). It is important to note that apoE is reduced to the same levels observed in the parental cell line after treatment with linoleic acid. This data reveals that PPARδ mediates the fatty acid repression of the apoE gene expression. The cells expressing the PPARδ antisense RNA (PPARδAS) also grew normally; however, when treated with PMA these cells did not differentiate (Fig 9A). Differentiation was not observed at any concentration of PMA (0.1 ng-25 ng/ml). Trypan blue staining of PPARδAS cells treated with PMA overnight revealed that the cells had died upon treatment (Fig 9B). There was no evidence for DNA condensation as judged by DAPI (4N,6-diamidino-2-phenylindole, Molecular Probes. Oregon) staining (data not shown). DNA fragmentation was not seen in agarose gel electrophoresis of genomic DNA prepared from these dead cells (data not shown). Therefore the PMA treated PPARδ-antisense cells do not appear to be dying by apoptosis. Using inhibitors for PKC and phospholipase A we have shown that we can block differentiation of parental THP-1 cells (Figure 10A); however, it appears that the PPARδ-antisense cells die in the presence of phorbol ester and these inhibitors (Figure 10B). This suggests that the cause of death is not downstream of PKC activation in the differentiation process.

We have shown that PPARδ is required for the inflammatory activation of macrophages (as evidenced by the activation of THP-1 cells), and that PEARδ promotes foam cell formation. The overexpression of PPARδ profoundly alters lipid storage in these cells and this is accomplished, in part, by altering the expression of the gene encoding apolipoprotein E. Inhibition (antagonism) of PPARδ activity can prevent foam cell formation and the development of drugs that are inhibitors of PPARδ activity are useful in the prevention and/or treatment of a large number of disorders that occur as a consequence of atherogenesis.

### Example 2: Lipid accumulation is promoted by an agonist for PPARδ.

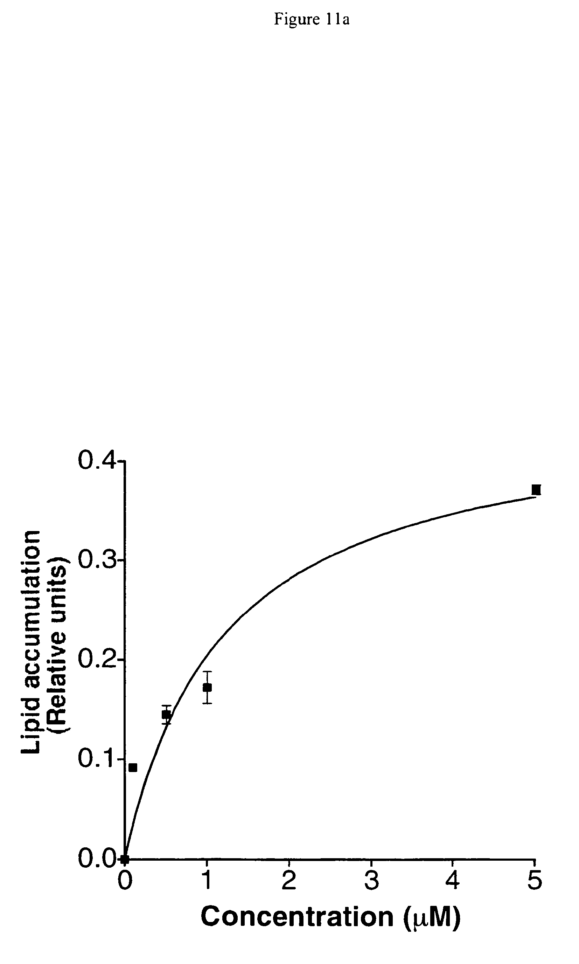

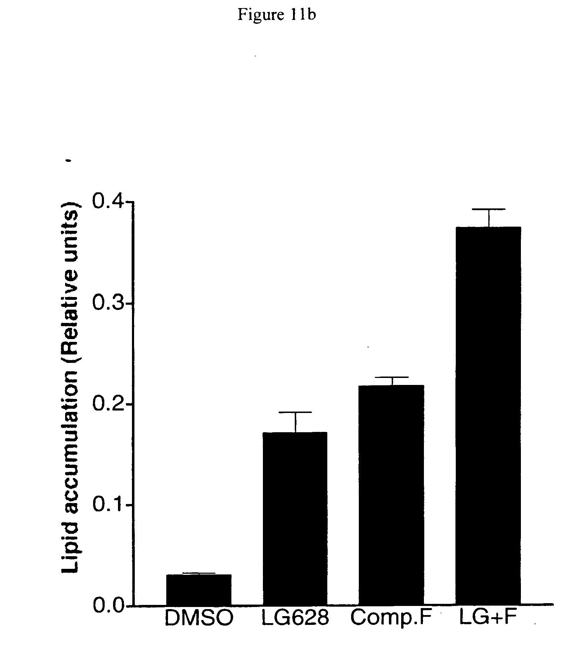

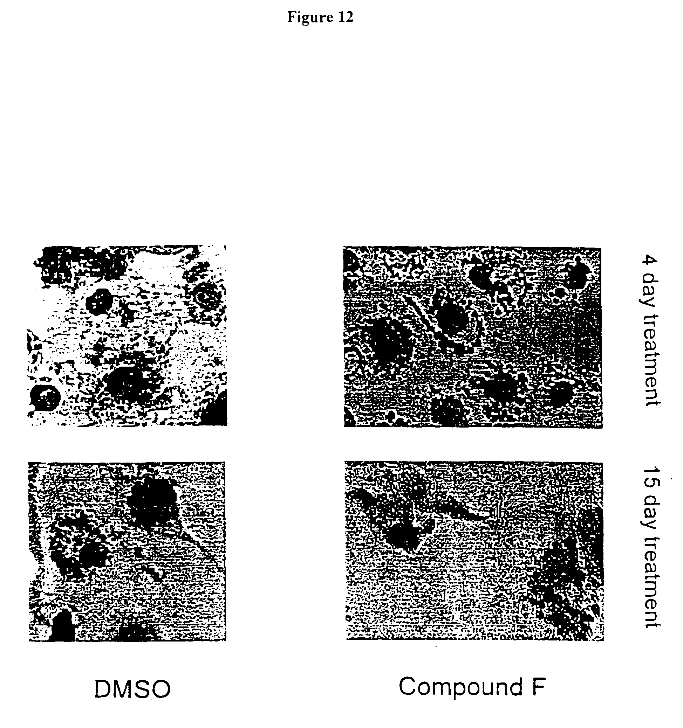

A PPARδ selective agonist (Compound F on page 9 of WO 97/28149) promotes lipid accumulation and promotes foam cell formation in the THP-1 assay described in Example 1. Although WO 97/28149 proposes using this PPARδ agonist (as well as other disclosed compounds) to treat cardiovascular disease, we have now shown that this compound promotes foam cell formation.. This effect is apparent at less than 500nM and is maximal at between 5 and 10 µM (see Figure 11A). Compound F co-operates with the RXR ligand LG100268 in the promotion of foam cell formation (see Figure 11B). This pharmacological activity is what we would predict from the studies described in Example 1. Compound F also promotes macrophage foam cell formation both in THP-1 cells (Figure 12) and primary human monocytes(sfigure 13).

### Example 3: Screening for inhibitors of PPARδ activity.

### Binding assays:

### Radioligand filtration assays

Recombinant hPPARδ is mixed with the radioactive ligand [³H]₂ L-783483 as described in WO 97/28149 and in Berger et al (1999) J. Biol. Chem. 274, 6718-6725 in the presence of a test compound. L-783483 has a K_{d} = 1nM. The mixture is applied to mixed cellulose filters and the fluid drawn through the filter by vacuum pressure. The radioactivity remaining on the filter will represent L-783483 bound to PPARδ. A decrease in the radioactivity bound will indicate the presence of a competing PPARδ compound. This assay determines binding properties of the test compound.

### Fluorescent fatty acid displacement.

The binding of *cis* or *trans* parinaric acid provides a fluorescent assay for PPAR ligands. "cis-Parinaric acid" is 9,15-cis, 11,13 *trans*-parinaric acid. "*trans-*Parinaric acid" is 9,11,13,15 *trans*-parinaric acid. Both are available from Molecular Probes, Oregon. Recombinant PPARδ is mixed with these fatty acids acids and a test compound would produce less fluorescence than observed in the presence of fatty acid/PPAR alone if significant binding of the test compound occurs. This assay determines whether the test compound binds PPARδ.
This assay would be automated easily by carrying it out in a multi-well format with an appropriate fluorimeter, giving a high throughput.

### Co-activator/receptor/lizand interaction assay (CARLA)

The binding of co-activator proteins, such as RIP140 or SRC-1, to PPARδ is dependent on the presence of a ligand. RIP140 and SRC-1 are proteins that bind PPARδ (krey *et al* (1997) *Mol. Endocrinol.* **11**, 779-791). Co-activators are translated *in vitro* in the presence of ³⁵S labelled methionine and mixed with purified recombinant hPPARδ in the presence of test compounds. The hPPAR will be immunoprecipitated and the precipitate will be analysed by SDS/PAGE followed by autoradiography, the presence of a radioactive signal corresponding to the co-receptor will indicate the presence of a PPARδ ligand. This assay may be used to identify co-activator binding that may prove to be antagonists; this is achieved by having known agonists present in each assay (see above).

### Functional assays.

### Transient transfection reporter construct assay

Cos-1 or CV-1 cells are transfected with an expression construct for PPARδ and a reporter construct with the luciferase under the control of a PPAR dependent enhancer. PPARδ-dependent enhancers include those derived from the acyl-CoA oxidase and cytochrome P4504A6 genes. This may be performed in a variety of multi-well culture formats and the transfected cells are treated with a known agonist of PPARδ and the test compounds. Cells are lysed *in situ* and assayed for luciferase activity in a luminometer. Luminometers are available that are compatible with high throughput screening. The assay described above examines the ability of the test compounds to block activity of a PPARδ agonist.

### Stable cell lines reporter construct assay.

Cell lines are transfected with constructs similar to those outlined above except that they also encode antibiotic resistance markers. This allows for the selection of cell lines that stably express PPARδ and contain the reporter sequences integrated into the genome. This cell line is grown and used for the detection of PPARδ activating compounds without the need for repetitive and costly transfections.

### Example 4: Foam cell assays

Foam cell assays are used to identify potential anti-atherogenic compounds. THP-1 cells in 96-well plate culture are treated with 0.1 to 10ng/ml PMA in order to induce macrophage differentiation. THP-1 cells are available as ECACC Accession No 88031201.

A source of fatty acid is supplied which is either serum (CPSR-3 serum replacement from Sigma) or 20-100µM linoleic acid.

Accumulation of fatty acid in the differentiating macrophage cells is measured in the presence or absence of a test compound after 3 to 7 days using Nile Red stain.

Test compounds which reduce the accumulation of fat compared to the absence of test compound are selected as potential anti-atherogenic agents or as lead compounds.

### Example 5: Prostate cancer cell tests

We have found that the PPARδ agonist, Compound F, can stimulate prostate epithelial growth. Both minimally transformed cells PNT1A and non-transforming prostate cancer cells (LNCaP) have growth stimulated by up to 40% by low concentrations of Compound F. This effect is also seen with the RXR ligand LG100268. Also, low concentrations of both Compound F and LG 100268 that do not significantly increase cell growth, stimulate cell growth when added together in the culture medium. The highly transformed prostate cancer cell line PC3 grows the fastest out of these lines and is not stimulated by Compound F.

We have transfected PC3 cells with expression constructs for PPARδantisense RNA; these have all failed to grow. In contrast, cell lines expressing PPARγ antisense RNA have been generated, which have altered but sustainable growth. In all experiments, cell lines containing the empty expression vector have been generated and have no phenotype. These experiments support the assertion that PPARδ is pro-proliferative and that PPARδ antagonists will be useful in the treatment of cancer.

Cell lines were plated at 2,500 cells per well in a 24-well plate and cultured in RPMI containing 5% dextran-coated charcoal-treated FCS. Increasing concentrations of Compound F or LG 100268 were included in the culture medium and the cultures were maintained for 7 days with changes of drugs and medium every 48 hours. The final cell number in each well was then determined by a colorimetric assay (Landegreen U. J. Immunol. Meth. 67, 379-388, (1984)) and the relative growth expressed as a percentage of the cell number in the control wells.

### REFERENCES

1. Issemann, I. & Green, S. (1990) "Activation of a member of the steroid hormone receptor superfamily by peroxisome proliferators" Nature 347, 645-50.
2. Dreyer, C. et al (1992) "Control of the peroxisomal beta-oxidation pathway by a novel family of nuclear hormone receptors" Cell 68, 879-87.
3. Palmer, C.N.A. et al (1998) "Peroxisome proliferator activated receptor-alpha expression in human liver" Molecular Pharmacology 53, 14-22.
4. Lee, S.S-T. et al (1995) "Targeted disruption of the alpha isoform of the peroxisome proliferator-activated receptor gene in mice results in abolishment of the pleiotropic effects of peroxisome proliferators" Mol. Cell. Biol. 15, 3012-3022.
5. Forman, B.M. et al (1995) "15-Deoxy-delta 12, 14-prostaglandin J2 is a ligand for the adipocyte determination factor PPAR gamma" Cell 83, 803-812.
6. Lehmann, J.M. et al (1995) "An anti-diabetic thiazolidinedione is a high affinity ligand for Peroxisome proliferator activated receptor γ" J. Biol. Chem. 270, 12953-12956.
7. Barak, Y. et al (1998) "PPARγ null mice - Early embryonic lethality due to a putative placental defect" Abstract No 303. Keystone Symposium on The Nuclear Receptor Gene Family. Incline Village, Nevada.
8. Schmidt, A. et al (1992) "Identification of a new member of the steroid hormone receptor superfamily that is activated by a peroxisome proliferator and fatty acids" Molecular Endocrinology 6, 1634-1641.
9. Saxena, U. et al (1992) "Lipoprotein lipase-mediated lipolysis of very low density lipoproteins increases monocyte adhesion to aortic endothelial cells" Biochemical & Biophysical Research Communications 189, 1653-1658.
10. Frostegard, J. et al (1990) "Oxidized low density lipoprotein induces differentiation and adhesion of human monocytes and the monocytic cell line U937" Proc. Nat. Acad. Sci. USA 87, 904-908.
11. Terkeltaub, R. et al (1994) "Oxidized LDL induces monocytic cell expression of interleukin-8, a chemokine with T-lymphocyte chemotactic activity" Arterioscler. Thromb. 14, 47-53.
12. Ross, R. (1993) "The pathogenesis of atherosclerosis; A perspective for the 1990s" Nature 362, 801.
13. Ricote, M. et al (1998) "The peroxisome proliferator activated receptor-γ is a negative regulator of macrophage activation" Nature 391, 79-82.
14. Jiang, C. et al (1998) "PPARγ agonists inhibit production of monocyte inflammatory cytokines" Nature 391, 82-86.
15. Marx, N. et al (1998) "Macrophages in human atheroma contain PPARγ" Am. J. Pathol. 153, 17-23.
16. Tontonoz, P. et al (1998) "PPARγ promotes monocyte/macrophage differentiation and uptake of oxidised LDL" Cell 93, 241-252.
17. Nagy, L. et al (1998) "Oxidised LDL regulates macrophage gene expression through ligand activation of PPARγ' Cell 93, 229-240.

## Claims

1. A composition for use in the treatment of atherosclerosis or diseases associated with atherosclerosis, heart disease, stroke, peripheral artery diseases, angina, restenosis and atheroma or a vascular disease selected from any one of atherosclerosis, heart disease, stroke or peripheral artery disease or for the treatment or prevention of cancer selected from skin cancer, breast cancer, colon cancer or prostate cancer, which composition comprises an inhibitor of PPARδ activity wherein said inhibitor of PPARδ activity is a nucleic acid-based inhibitor of PPARδ activity in a cell being a PPARδ-selective antisense molecule or a PPARδ-selective ribozyme.

2. Use of an inhibitor of PPARδ activity in the manufacture of a medicament for the treatment of atherosclerosis or diseases associated with atherosclerosis, heart disease, stroke, peripheral artery diseases, angina, restenosis and atheroma or a vascular disease selected from any one of atherosclerosis, heart disease, stroke or peripheral artery disease or for the treatment or prevention of cancer selected from skin cancer, breast cancer, colon cancer or prostate cancer wherein the said inhibitor is a nucleic acid-based inhibitor of PPARδ activity in a cell being a PPARδ-selective antisense molecule or a PPARδ-selective ribozyme.

## Patentansprüche

1. Zusammensetzung zur Verwendung bei der Behandlung von Arteriosklerose oder Erkrankungen, die mit Arteriosklerose in Zusammenhang stehen, Herzerkrankung, Schlaganfall, Erkrankungen der peripheren Arterien, Angina, Restenose und Atherom oder einer Gefäßerkrankung aus der Gruppe Arteriosklerose, Herzerkrankung, Schlaganfall oder periphere Gefäßerkrankung oder zur Behandlung oder Prävention von Krebs aus der Gruppe Hautkrebs, Brustkrebs, Kolonkrebs oder Prostatakrebs, wobei die Zusammensetzung einen Inhibitor der PPARδ-Aktivität enthält und es sich bei dem Inhibitor der PPARδ-Aktivität um einen auf einer Nucleinsäure basierenden Inhibitor der PPARδ-Aktivität in einer Zelle handelt, der entweder ein PPARδ-selektives Antisense-Molekül oder ein PPARδ-selektives Ribozym darstellt.

2. Verwendung eines Inhibitors der PPARδ-Aktivität für die Herstellung eines Medikaments zur Behandlung von Arteriosklerose oder Erkrankungen, die mit Arteriosklerose in Zusammenhang stehen, Herzerkrankung, Schlaganfall, Erkrankungen der peripheren Arterien, Angina, Restenose und Atheroma oder einer Gefäßerkrankung aus der Gruppe Arteriosklerose, Herzerkrankung, Schlaganfall oder periphere Gefäßerkrankung oder zur Behandlung oder Prävention von Krebs aus der Gruppe Hautkrebs, Brustkrebs, Kolonkrebs oder Prostatakrebs, wobei es sich bei dem Inhibitor um einen auf einer Nucleinsäure basierenden Inhibitor der PPARδ-Aktivität in einer Zelle handelt, der entweder ein PPARδ-selektives Antisense-Molekül oder ein PPARδselektives Ribozym darstellt.

## Revendications

1. Composition destinée à être utilisée dans le traitement de l'athérosclérose ou de maladies associées à l'athérosclérose, d'une maladie cardiaque, d'un ictus, de maladies des artères périphériques, de l'angine de poitrine, d'une resténose et d'un athérome, ou d'une maladie vasculaire choisie parmi n'importe lesquelles des maladies consistant en l'athérosclérose, une maladie cardiaque, un ictus et une maladie des artères périphériques, ou dans le traitement ou la prévention d'un cancer choisi entre le cancer de la peau, le cancer du sein, le cancer du côlon et le cancer de la prostate, composition qui comprend un inhibiteur d'activité de PPARδ, dans laquelle ledit inhibiteur d'activité de PPARδ est un inhibiteur à base d'acide nucléique d'activité de PPARδ dans une cellule qui est une molécule antisens sélective vis-à-vis de PPARδ ou bien un ribozyme sélectif vis-à-vis de PPARδ.

2. Utilisation d'un inhibiteur d'activité de PPARδ dans la production d'un médicament destiné au traitement de l'athérosclérose ou de maladies associées à l'athérosclérose, d'une maladie cardiaque, d'un ictus, de maladies des artères périphériques, de l'angine de poitrine, d'une resténose et d'un athérome, ou d'une maladie vasculaire choisie parmi n'importe lesquelles des maladies consistant en l'athérosclérose, une maladie cardiaque, un ictus et une maladie des artères périphériques, ou pour le traitement ou la prévention d'un cancer choisi entre le cancer de la peau, le cancer du sein, le cancer du côlon et le cancer de la prostate, dans laquelle ledit inhibiteur est un inhibiteur à base d'acide nucléique d'activité de PPARδ dans une cellule qui est une molécule antisens sélective vis-à-vis de PPARδou un ribozyme sélectif vis-à-vis de PPARδ.
